# EUROPEAN PATENT SPECIFICATION

(11) **EP 4 218 841 B1**
(45) Date of publication and mention of the grant of the patent: **01.07.2026**
(21) Application number: 21871612.4
(22) Date of filing: 24.09.2021
(51) Int. Cl.: C25D 11/26, C25D 11/00, A61L 27/06, A61L 27/50, A61L 27/30, A61L 27/04, A61C 8/00, A61F 2/28

(54) **BONE IMPLANT HAVING POROUS MEMBRANE, AND PRODUCTION METHOD THEREFOR**
KNOCHENIMPLANTAT MIT PORÖSER MEMBRAN UND HERSTELLUNGSVERFAHREN DAFÜR
IMPLANT OSSEUX AYANT UNE MEMBRANE POREUSE, ET SA MÉTHODE DE PRODUCTION

(30) Priority: 25.09.2020 CN 202011025357
(43) Date of publication of application: 02.08.2023
(73) Proprietor: Beijing Huayu Chuangxin Technologies Co., Ltd., Beijing 101407 (CN); Beijing Huayu Chuangxin TA-NB Science & Technology Co., Ltd., Beijing 101407 (CN)
(72) Inventor: LIU, Yuzhong, Beijing 101407 (CN)
(74) Representative: Herzog IP Patentanwalts GmbH
(86) International application number: PCT/CN2021/120407
(87) International publication number: WO 2022/063243

(56) References cited:
- EP-A1- 3 195 825
- WO-A1-2016/042515
- WO-A1-2019/008552
- CN-A- 1 712 566
- CN-A- 101 311 329
- CN-A- 101 686 861
- CN-A- 101 720 209
- CN-A- 109 487 323
- CN-A- 85 101 505
- CN-A- 86 102 269
- JP-A- S62 230 997
- NA WANG ET AL: "Study on the Anticorrosion, Biocompatibility, and Osteoinductivity of Tantalum Decorated with Tantalum Oxide Nanotube Array Films", APPLIED MATERIALS & INTERFACES, vol. 4, no. 9, 26 September 2012 (2012-09-26), US, pages 4516 - 4523, XP055393829, ISSN: 1944-8244, DOI: 10.1021/am300727v
- VILARINHO PAULA MARIA ET AL: "Are lithium niobate (LiNbO3) and lithium tantalate (LiTaO3) ferroelectrics bioactive?", MATERIALS SCIENCE AND ENGINEERING C, vol. 39, 20 March 2014 (2014-03-20), pages 395 - 402, XP029029228, ISSN: 0928-4931, DOI: 10.1016/J.MSEC.2014.03.026

## Description

### Technical Field

The present invention relates to a bone implant with a porous membrane and a method for preparing the bone implant.

### Background Art

Metal materials have excellent comprehensive mechanical properties and anti-fatigue properties and are particularly suitable for use as orthopedic implant materials. With the rapid development of materials science, orthopedic implant materials have experienced the development process from stainless steel, cobalt-chromium alloys to titanium alloys and achieved relatively good effects. However, complicated environments inside human bodies cause material corrosion, which results in a release of toxic elements and further a reduction in biocompatibility of some metal materials. In addition, materials in the prior art, such as stainless steel, cobalt-chromium alloys and titanium alloys, have a relatively dark color or even are black, which affects the aesthetic properties of the materials and limits the scope of the applications. The above deficiencies have certain negative impacts on the application of the metal materials as biomedical materials.

Porous tantalum is a relatively ideal orthopedic implant material that has emerged in recent years. As a refractory metal, it has a melting point of nearly 3000°C, is dark gray in appearance and has a smooth surface. Compared with existing medical metal materials, tantalum has two main advantages: (1) tantalum has more excellent corrosion resistance; (2) tantalum has better biocompatibility and porous tantalum has a relatively low elasticity modulus. However, due to its dark gray color, tantalum affects aesthetic appearance when used particularly in dental implants.

Porous tantalum is a relatively ideal orthopedic implant material, and documented orthopedic materials made of tantalum have an elasticity modulus between the elasticity modulus of cortical bone and the elasticity modulus of cancellous bone. However, the elasticity modulus of porous tantalum is closer to that of human cancellous bone, but significantly lower than that of cortical bone. Moreover, a bone tissue that bears the load of a human body is mainly cortical bone. From the perspective of animal experiments and clinical application effects, the elasticity modulus of bone implants should be closer to that of human cortical bone; a too high elasticity modulus makes it easy to produce stress shielding while a too low elasticity modulus is not conducive to force transmission. The present invention can provide elasticity modulus from low to high for selection under different coating conditions.

However, with regard to dental implants, from a biomechanical point of view, the elasticity modulus of a dental implant has an effect on stress distribution at a bone interface. Generally, the higher the elasticity modulus of a dental implant, the smaller the intemal stress of the bone around the neck and the greater the internal stress of the bone at the root; the lower the elasticity modulus of a dental implant, the greater the relative displacement motion of the dental implant and the bone interface. When the elasticity modulus of a dental implant is similar to that of cortical bone, cancellous bone, the biomechanical compatibility is relatively good. Some scholars have suggested that a high elasticity modulus is better and that an elasticity modulus suitable for a dental implant is preferably 70 to 200 GPa, and it is reported that the elasticity modulus of cortical bone is about 18 GPa. However, there are different requirements for the elasticity modulus of cranium (skull) implants. Dental implants are made of titanium alloys or other materials into cylinders or cones, on the outer surfaces of which threads are formed, with an aim that human bones can grow into the threads, which allows the implants to integrate with the human bones and allows for the enhanced compatibility and firmness of the implants and the human bodies. Nevertheless, threaded ribs are limited in depth, and an excessive depth affects the strength of the implants.

Na Wang et al. « Study on Anticorrosion, Biocompatibility and Osteoinductivity of Tantalum decorated with Tantalom Oxide Nanotube array Films", Applied Materials & Interfaces, Vol. 4 (9), pp. 4516-4523 relates to anodized tantalum implants.

WO 2016/042515 A1 relates to dental implants comprising a titanium body and a coating formed by a tantalum deposit.

CN85101505 A relates to an article made of tantalum or niobium or an alloy therof, having a thin film on its surface.

Therefore, there is still a need to seek a bone implant with aesthetic appearance and/or higher biocompatibility, particularly a dental implant and a cranium (skull) implant.

### Contents of Invention

For this purpose, the present invention provides a bone implant comprising:
(1) a substrate; and
(2) a porous membrane on the substrate, wherein one of these features applies:
   i) the substrate is a tantalum substrate and the porous membrane is a porous lithium tantalate membrane,
   ii) the substrate is a niobium substrate and the porous membrane is a porous lithium niobate membrane, or
   iii) the substrate is a tantalum-niobium alloy substrate and the porous membrane is a porous lithium tantalate-lithium niobate mixture membrane.

Further, the present invention provides a bone implant comprising:
(1) a tantalum substrate, a niobium substrate or a tantalum-niobium alloy substrate; and
(2) a porous lithium tantalate membrane on the tantalum substrate, wherein the lithium tantalate membrane contains a tantalum oxide,
   a porous lithium niobate membrane on the niobium substrate, wherein the lithium niobate membrane contains a niobium oxide,
   a porous lithium tantalate-lithium niobate mixture membrane on the tantalum-niobium alloy substrate, wherein the mixture membrane contains a tantalum oxide and a niobium oxide.

The present invention also provides a method for preparing the aforementioned bone implant, comprising:
(1) providing a substrate; and
(2) forming a porous membrane on the substrate, wherein one of these features applies:
   (i) the substrate is a tantalum substrate and the porous membrane is a porous lithium tantalate membrane, which contains a tantalum oxide,
   (ii) the substrate is a niobium substrate and the porous membrane is a porous lithium niobate membrane, which contains a niobium oxide or
   (iii) the substrate is a tantalum-niobium alloy substrate and the porous membrane is a porous lithium tantalate-lithium niobate mixture membrane, which contains a tantalum oxide and a niobium oxide.

The present invention also relates to a use of the bone implant in medical materials, such as dental implants.

The bone implant of the present invention has one or more of the following beneficial effects: (1) The metal compound membrane provided is porous, which is conducive to guiding a human bone into the implant and enhancing the compatibility and firmness of the implant. Meanwhile, the compound membrane layer seems to be close to a ceramic body, which has the effect of insulation or heat insulation and reduces the stimulation of rapid heat to a human body. (2) The membrane layer is not formed by ion deposition in a solution, but results from the participation of the metal of the implant in an electrochemical reaction; there is a performance transition layer; the membrane layer is firmly bonded to the substrate, and the bone implant has excellent corrosion resistance. (3) The elasticity modulus of the bone implant can be adjusted according to process conditions so that it has higher biocompatibility with the elasticity modulus of a human or animal bone (such as an alveolar bone). (4) The white color of the bone implant is close to the color of the bone itself and the bone implant has an aesthetic appearance. (5) The bone implant has excellent bacteriostatic properties since the membrane layer results from coating treatment at high temperatures (above 400°C).

### Description of Figures

Figures 1a-1c show SEM photographs of a pure tantalum flake not coated with a porous lithium tantalate membrane.
Figures 2a-2d show SEM photographs of a tantalum flake coated with a porous lithium tantalate membrane according to one embodiment of the present invention.
Figures 3a-3c show SEM photographs of a tantalum flake coated with a porous lithium tantalate membrane according to one embodiment of the present invention.
Figures 4a-4c show SEM photographs of a tantalum flake coated with a porous lithium tantalate membrane according to one embodiment of the present invention.
Figures 5a-5c show SEM photographs of a tantalum flake coated with a porous lithium tantalate membrane according to one embodiment of the present invention.
Figures 6a-6c show SEM photographs of a tantalum flake coated with a porous lithium tantalate membrane according to one embodiment of the present invention.
Figures 7a-7d show SEM photographs of a tantalum flake coated with a porous lithium tantalate membrane according to one embodiment of the present invention.
Figures 8a-8c show SEM photographs of a tantalum flake coated with a porous lithium tantalate membrane according to one embodiment of the present invention.
Figure 9 shows an appearance photograph of a tantalum flake coated with a porous lithium tantalate membrane according to one embodiment of the present invention.
Figure 10 shows an appearance photograph of a tantalum flake coated with a porous lithium tantalate membrane according to one embodiment of the present invention.
Figure 11 shows an appearance photograph of a tantalum flake coated with a porous lithium tantalate membrane according to one embodiment of the present invention.
Figure 12 shows an appearance photograph of a tantalum flake coated with a porous lithium tantalate membrane according to one embodiment of the present invention.
Figure 13 shows an appearance photograph of a tantalum flake and a tantalum dental implant coated with a porous lithium tantalate membrane according to one embodiment of the present invention.
Figure 14 shows an appearance photograph of a tantalum flake and a tantalum dental implant coated with a porous lithium tantalate membrane according to one embodiment of the present invention.
Figure 14a shows an appearance photograph of a porous tantalum dental implant coated with a porous lithium tantalate membrane according to one embodiment of the present invention.
Figures 15a-15g show appearance photographs of a tantalum flake coated with a porous lithium tantalate membrane according to one embodiment of the present invention.
Figures 16a-16b show SEM photographs of a tantalum flake coated with a porous lithium tantalate membrane according to one embodiment of the present invention.
Figures 17a-17b show SEM photographs of a tantalum flake coated with a porous lithium tantalate membrane according to one embodiment of the present invention.
Figures 18a-18b show SEM photographs of a tantalum flake coated with a porous lithium tantalate membrane according to one embodiment of the present invention.
Figures 19a-19b show SEM photographs of a tantalum flake coated with a porous lithium tantalate membrane according to one embodiment of the present invention.
Figures 20a-20b show SEM photographs of a tantalum flake coated with a porous lithium tantalate membrane according to one embodiment of the present invention.
Figures 21a-21b show SEM photographs of a tantalum flake coated with a porous lithium tantalate membrane according to one embodiment of the present invention.
Figures 22a-22b show SEM photographs of a tantalum flake coated with a porous lithium tantalate membrane according to one embodiment of the present invention.
Figures 23a-23i show appearance photographs of a tantalum flake coated with a porous lithium tantalate membrane according to one embodiment of the present invention.
Figures 24a-24b show SEM photographs of a tantalum flake coated with a porous lithium tantalate membrane according to one embodiment of the present invention.
Figures 25a-25b show SEM photographs of a tantalum flake coated with a porous lithium tantalate membrane according to one embodiment of the present invention.
Figures 26a-26b show SEM photographs of a tantalum flake coated with a porous lithium tantalate membrane according to one embodiment of the present invention.
Figures 27a-27b show SEM photographs of a tantalum flake coated with a porous lithium tantalate membrane according to one embodiment of the present invention.
Figures 28a-28b show SEM photographs of a tantalum flake coated with a porous lithium tantalate membrane according to one embodiment of the present invention.
Figures 29a-29b show SEM photographs of a tantalum flake coated with a porous lithium tantalate membrane according to one embodiment of the present invention.
Figures 30a-30b show SEM photographs of a tantalum flake coated with a porous lithium tantalate membrane according to one embodiment of the present invention.
Figures 31a-31b show SEM photographs of a tantalum flake coated with a porous lithium tantalate membrane according to one embodiment of the present invention.
Figures 32a-32b show SEM photographs of a tantalum flake coated with a porous lithium tantalate membrane according to one embodiment of the present invention.
Figure 33a shows an appearance photograph of a tantalum cranium coated with a porous lithium tantalate membrane according to one embodiment of the present invention.
Figure 33b shows a schematic cross-sectional diagram of a tantalum cranium coated with a porous lithium tantalate membrane according to one embodiment of the present invention.
Figure 33c shows a schematic top view diagram of a tantalum cranium coated with a porous lithium tantalate membrane according to one embodiment of the present invention.
Figure 34 shows an appearance photograph of a tantalum-niobium alloy flake coated with a porous lithium tantalate-lithium niobate mixture membrane according to one embodiment of the present invention.
Figures 35a-35b show SEM photographs of a tantalum-niobium alloy flake coated with a porous lithium tantalate-lithium niobate mixture membrane according to one embodiment of the present invention.
Figure 36 shows an appearance photograph of a tantalum-niobium alloy flake coated with a porous lithium tantalate-lithium niobate mixture membrane according to one embodiment of the present invention.
Figures 37a-37b show SEM photographs of a tantalum-niobium alloy flake coated with a porous lithium tantalate-lithium niobate mixture membrane according to one embodiment of the present invention.
Figure 38 shows an appearance photograph of a niobium flake coated with a porous lithium niobate membrane according to one embodiment of the present invention.
Figures 39a-39b show SEM photographs of a niobium flake coated with a porous lithium niobate membrane according to one embodiment of the present invention.
Figure 40 shows an appearance photograph of a niobium flake coated with a porous lithium niobate membrane according to one embodiment of the present invention.
Figure 41 shows an appearance photograph of a niobium cranium coated with a porous lithium niobate membrane according to one embodiment of the present invention.
Figures 42a-42b show SEM photographs of a niobium flake coated with a porous lithium niobate membrane according to one embodiment of the present invention.
Figure 43 shows an appearance photograph of a niobium flake coated with a porous lithium niobate membrane according to one embodiment of the present invention.
Figures 44a-44b show SEM photographs of a niobium flake coated with a porous lithium niobate membrane according to one embodiment of the present invention.

### Specific Embodiments

In one embodiment, the present invention provides a bone implant comprising:
(1) a tantalum substrate; and
(2) a porous lithium tantalate membrane on the tantalum substrate, wherein the lithium tantalate membrane contains a tantalum oxide.

In one embodiment, the present invention provides a bone implant comprising:
(1) a niobium substrate; and
(2) a porous lithium niobate membrane on the niobium substrate, wherein the lithium niobate membrane contains a niobium oxide.

In one embodiment, the present invention provides a bone implant comprising:
(1) a tantalum-niobium alloy substrate; and
(2) a porous lithium tantalate-lithium niobate mixture membrane on the tantalum-niobium alloy substrate, wherein the mixture membrane contains a tantalum oxide and a niobium oxide.

In one embodiment, the bone implant comprises: (1) a tantalum substrate; and (2) a porous lithium tantalate membrane on at least a portion of the surface of the tantalum substrate, wherein the porous lithium tantalate membrane has a pore diameter of 0.1-1 µm. For crack-like pores, the length thereof can reach about 40 µm.

In one embodiment, the bone implant comprises: (1) a niobium substrate; and (2) a porous lithium niobate membrane on at least a portion of the surface of the niobium substrate, wherein the porous lithium niobate membrane has a pore diameter of 0.1-1 µm. For crack-like pores, the length thereof can reach about 40 µm.

In one embodiment, the bone implant comprises: (1) a tantalum-niobium alloy substrate; and (2) a porous lithium tantalate-lithium niobate mixture membrane on at least a portion of the surface of the tantalum-niobium alloy substrate, wherein the mixture membrane has a pore size of 0.1-1 µm. For crack-like pores, the length thereof can reach about 40 µm.

In the context of the present invention, the pores are not limited to round pores, but may be pores with irregular shapes, e.g., crack-like pores.

In one embodiment of the present invention, the bone implant has a porosity of 30% or more, such as a porosity of 50-80% or a porosity of 60-90%.

Examples of the tantalum substrate suitable for the present invention include a pure tantalum substrate and a tantalum alloy substrate, wherein the tantalum alloy substrate is preferably a tantalum-niobium alloy substrate.

In one embodiment of the present invention, the bone implant, such as a dental implant, has an elasticity modulus of at least 150 GPa.

In another embodiment of the present invention, the bone implant, such as a dental implant, has an elasticity modulus of at least 170 GPa.

In yet another embodiment of the present invention, the bone implant, such as a dental implant, has an elasticity modulus of 150-200 GPa.

In still another embodiment of the present invention, the bone implant, such as a dental implant, has an elasticity modulus of 170-190 GPa.

In one embodiment of the present invention, the bone implant, such as a cranium (skull) implant, has an elasticity modulus of 10-160 GPa, as measured by nanoindentation.

In another embodiment of the present invention, the bone implant, such as a cranium (skull) implant, has an elasticity modulus of 20-150 GPa, as measured by nanoindentation.

In yet another embodiment of the present invention, the bone implant, such as a cranium (skull) implant, has an elasticity modulus of 30-140 GPa, as measured by nanoindentation.

In yet another embodiment of the present invention, the bone implant, such as a cranium (skull) implant, has an elasticity modulus of 40-130 GPa, as measured by nanoindentation.

In still another embodiment of the present invention, the bone implant, such as a cranium (skull) implant, has an elasticity modulus of 50-120 GPa, as measured by nanoindentation.

In still another embodiment of the present invention, the bone implant, such as a cranium (skull) implant, has an elasticity modulus of 60-110 GPa, as measured by nanoindentation.

In still another embodiment of the present invention, the bone implant, such as a cranium (skull) implant, has an elasticity modulus of 70-100 GPa, as measured by nanoindentation.

In still another embodiment of the present invention, the bone implant, such as a cranium (skull) implant, has an elasticity modulus of 80-90 GPa, as measured by nanoindentation.

In one embodiment of the present invention, the entire surface of the tantalum substrate is covered with a porous lithium tantalate membrane.

In one embodiment of the present invention, the entire surface of the niobium substrate is covered with a porous lithium niobate membrane.

In one embodiment of the present invention, the entire surface of the tantalum-niobium alloy substrate is covered with a porous lithium tantalate-lithium niobate mixture membrane.

From a biomechanical point of view, the elasticity modulus of a dental implant has an effect on stress distribution at a bone interface. Generally, the higher the elasticity modulus of a dental implant, the smaller the internal stress of the bone around the neck and the greater the internal stress of the bone at the root; the lower the elasticity modulus of a dental implant, the greater the relative displacement motion of the dental implant and the bone interface. When the elasticity modulus of a dental implant is similar to that of cortical bone, cancellous bone, the biomechanical compatibility is relatively poor. A suitable elasticity modulus of a dental implant is 70 GPa or more. The bone implants of the present invention, including dental implants, have an elasticity modulus of at least 150 GPa. Therefore, the dental implant of the present invention has a very suitable elasticity modulus and an excellent biomechanical compatibility.

In one embodiment of the present invention, examples of the bone implant include bone prostheses, such as cranium and dental implants.

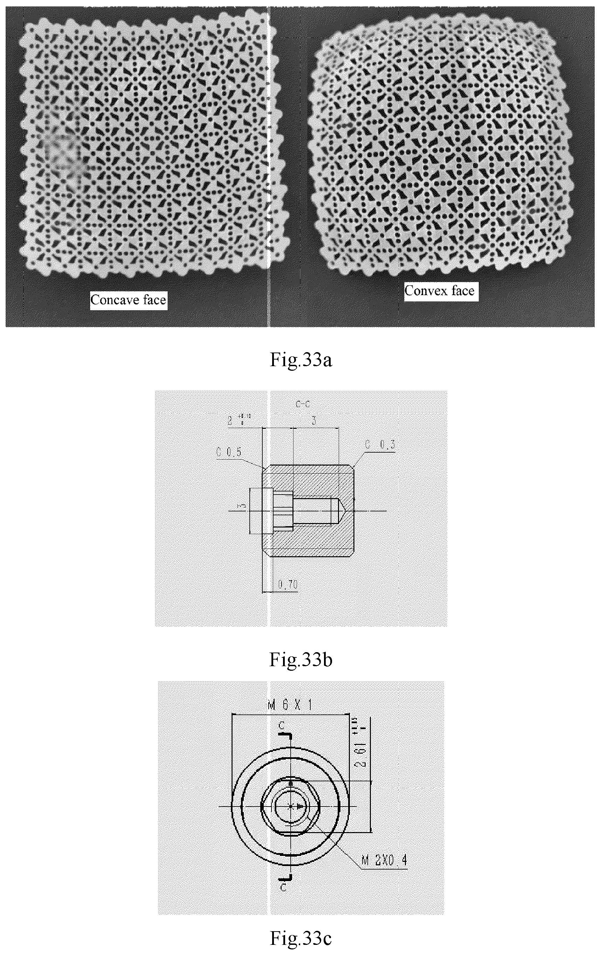

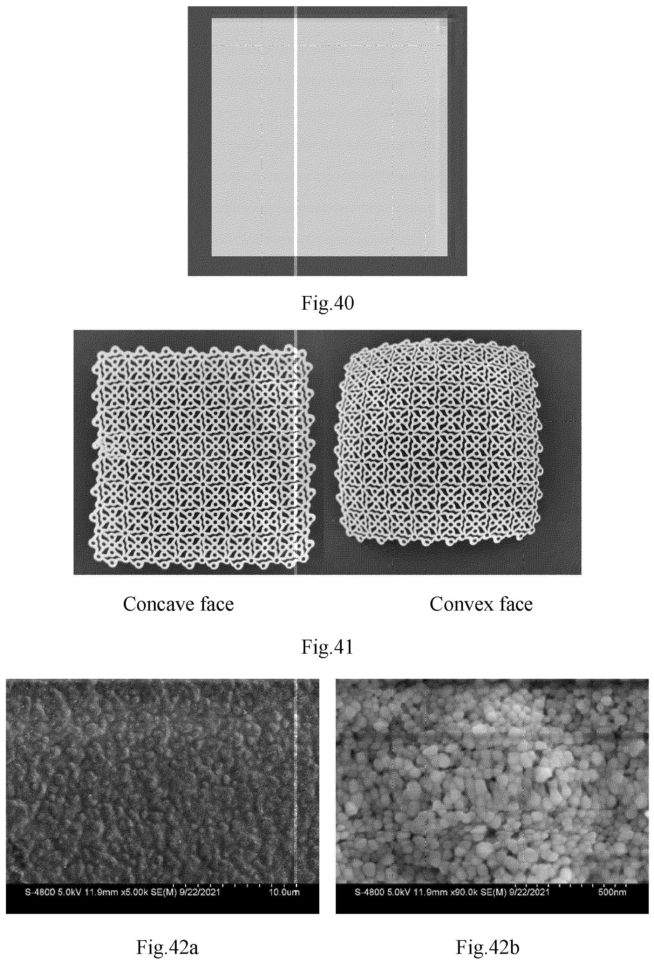

In one embodiment of the present invention, the cranium is porous, such as the tantalum cranium shown in Figure 33a and the niobium cranium shown in Figure 41.

In one embodiment of the present invention, the dental implant comprises a denture abutment, a main body and a neck connecting the denture abutment and the main body. The method for the preparation of the dental implant is known to those skilled in the art, and reference can be made to, for example, CN109758245A, CN109965996A and CN110610046A.

In a preferred embodiment of the present invention, the dental implant comprises a denture abutment, a main body and a neck connecting the denture abutment and the main body, wherein the main body is provided with external threads in contact with the alveolar bone on the outer surface thereof.

In one embodiment of the present invention, the bone implant has a similar color to the bone. In a preferred embodiment of the present invention, the bone implant is white, particularly for the denture abutment.

In one embodiment of the present invention, the substrate is porous. The porous substrate has excellent mechanical properties and tissue compatibility. Human tissue reconstruction, bone grafting, replacement and the like can all be realized by implanting a porous material into a bone-deficient part; the bone grows into a host interface along porous pores so that the porous material is bound fully and tightly and integrated with a bone tissue. After being implanted into a human body as a support material, the porous material does not need to degrade in the human body due to its excellent tissue compatibility, so there is no need to remove it by a secondary surgery. A porous metal is solid in texture and superior to a spongy bone, a ceramic product and a freeze-dried bone chip in respect of both wear resistance and fatigue resistance, and can provide sufficient physiological load.

Tantalum, niobium, tantalum-niobium alloys and titanium are refractory metals, and the abovementioned porous metals are generally prepared by the methods for the preparation of refractory metals. For example, porous tantalum can be prepared either by powder sintering by a known method or by a vapor deposition method. Other methods for preparing porous tantalum in the art can also be used.

In one embodiment of the present invention, the tantalum used is dense. Prior art documents all use porous tantalum as a medical orthopedic material and are silent about the use of a dense tantalum metal as a medical orthopedic material. With regard to dental implants, from a biomechanical point of view, the elasticity modulus of a dental implant has an effect on stress distribution at a bone interface. Generally, the higher the elasticity modulus of a dental implant, the smaller the internal stress of the bone around the neck and the greater the internal stress of the bone at the root; the lower the elasticity modulus of a dental implant, the greater the relative displacement motion of the dental implant and the bone interface. When the elasticity modulus of a dental implant is similar to that of cortical bone, cancellous bone, the biomechanical compatibility is relatively poor. A suitable elasticity modulus of a dental implant is 70 GPa or more. Since a dental implant often assumes the function of chewing and carries a very heavy load, the tensile strength and elasticity modulus of porous tantalum cannot meet the load requirement. The dense metal tantalum is relatively high in terms of tensile strength and elasticity modulus and is more suitable for use in a dental implant.

One embodiment of the present invention is to coat a porous tantalum dental implant with a layer of porous lithium tantalate membrane, thereby improving the mechanical properties, such as tensile strength and elasticity modulus, of the dental implant.

One embodiment of the present invention is to coat a porous niobium dental implant with a layer of porous lithium niobate membrane, thereby improving the mechanical properties, such as tensile strength and elasticity modulus, of the dental implant.

One embodiment of the present invention is to coat a porous tantalum niobium dental implant with a layer of porous lithium tantalate-lithium niobate mixture membrane, thereby improving the mechanical properties, such as tensile strength and elasticity modulus, of the dental implant.

Another embodiment of the present invention is to coat dense metal tantalum with a layer of porous lithium tantalate membrane to allow its tensile strength and elasticity modulus to become higher, which makes it possible to better meet the requirements for the tensile strength and elasticity modulus of the dental implant. Moreover, the tantalum substrate has a porous lithium tantalate membrane on its surface so that the dental implant can be better compatible with human biological tissues, which makes it possible that human biological tissues can be bound with the tantalum substrate through the porous lithium tantalate membrane, and makes it possible that the tantalum material can be bound fully and more tightly and integrated with human tissues, such as alveolar bone.

In one embodiment of the present invention, it is possible that a non-exposed part of a bone implant, such as a dental implant, is not coated with a layer of lithium tantalate membrane, but is made using only a pure tantalum material. The denture abutment and the neck are preferably coated with a lithium tantalate membrane to increase aesthetic appearance.

The methods for the preparation of a dense tantalum material are known to those skilled in the art, e.g., it can be obtained by such methods as casting and rolling.

In one embodiment of the present invention, the method for preparing the bone implant comprises: (1) providing a tantalum substrate; and (2) forming a layer of porous lithium tantalate membrane on the tantalum substrate.

In one embodiment of the present invention, the method for preparing the bone implant comprises: (1) providing a niobium substrate; and (2) forming a layer of porous lithium niobate membrane on the niobium substrate.

In one embodiment of the present invention, the method for preparing the bone implant comprises: (1) providing a tantalum-niobium alloy substrate; and (2) forming a layer of porous lithium tantalate-lithium niobate mixture membrane on the tantalum-niobium alloy substrate.

In one embodiment of the present invention, the substrate has a shape desired for the bone implant, e.g., the shapes of a dental implant and a cranium.

In a preferred embodiment of the present invention, a tantalum substrate having a desired bone implant shape is coated with a layer of porous lithium tantalate membrane.

In another embodiment of the present invention, a tantalum substrate having a desired bone implant shape is coated with a layer of porous lithium tantalate membrane; moreover, the unwanted porous lithium tantalate membrane on the bone implant is removed by cutting or grinding, and the porous lithium tantalate membrane is retained only on a portion of the surface of the bone implant. Concretely speaking, a plurality of unions manufactured, which are qualified in appearance and size and whose inner pores have not been processed, are coated with a lithium tantalate membrane, and then the inner pores are processed; in this way, the inner pores can be kept uncoated.

In a preferred embodiment of the present invention, a tantalum substrate having a desired bone implant shape is coated with a layer of porous lithium tantalate membrane on a portion of the surface thereof.

In one embodiment of the present invention, the porous membrane has a thickness of 1-20 µm, preferably 2-10 µm, more preferably 3-5 µm.

In step (2), the porous lithium tantalate membrane is formed by a molten salt electrochemical method.

In one embodiment of the present invention, in step (2), a tantalum substrate is placed in an oxygen-containing inorganic lithium salt (such as LiNO₃) or a mixed melt of an oxygen-containing inorganic lithium salt and lithium hydroxide, or a mixed molten liquid of a salt and lithium hydroxide or a mixed molten liquid of a lithium salt and an oxygen-containing salt, at a temperature of 250°C to 650°C, and an anode voltage of 1 to 66V is applied, which voltage is maintained for 0.01 to 200 hours, with a boosting current density of 1 to 1000 mA/cm², to form a porous lithium tantalate membrane.

In another embodiment of the present invention, in step (2), a tantalum substrate is placed in an oxygen-containing inorganic lithium salt (such as LiNO₃) or a mixed melt of an oxygen-containing inorganic lithium salt and lithium hydroxide, or a mixed molten liquid of a salt and lithium hydroxide or a mixed molten liquid of a lithium salt and an oxygen-containing salt, at a temperature of 440°C to 600°C, and an anode voltage of 10 to 30V is applied, which voltage is maintained for 5 minutes to 10 hours, with a boosting current density of 1 to 1000 mA/cm², to form a porous lithium tantalate membrane.

In yet another embodiment of the present invention, in step (2), a tantalum substrate is placed in an oxygen-containing inorganic lithium salt (such as LiNO₃) or a mixed melt of an oxygen-containing inorganic lithium salt and lithium hydroxide, or a mixed molten liquid of a salt and lithium hydroxide or a mixed molten liquid of a lithium salt and an oxygen-containing salt, at a temperature of 570°C to 598°C, and an anode voltage of 10 to 20V is applied, which voltage is maintained for 8 to 30 minutes, with a boosting current density of 5 to 20 mA/cm², to form a porous lithium tantalate membrane.

In a preferred embodiment, in step (2), an ultrasonic generator can be placed in the mixed melt or the mixed molten liquid.

Preferably, the molten salt electrochemical method in step (2) is a molten lithium salt electrochemical method.

Before step (2), the step of anodizing a tantalum substrate can be carried out.

In the anodizing step, a Ta₂O₅ membrane, such as an amorphous Ta₂O₅ membrane, is formed on the tantalum substrate. Those without pores are suitable for use on tantalum capacitors.

A Ta₂O₅ membrane may also be porous, which is more advantageous for the preparation of a porous lithium tantalate membrane. Specifically, an implant is placed in concentrated sulfuric acid at a temperature of 190°C to 245°C (i.e., a porous Ta₂O₅ membrane is formed in 98% concentrated sulfuric acid).

In one embodiment of the present invention, a layer of amorphous Ta₂O₅ membrane is formed on a tantalum substrate by anodic oxidation in the anodizing step. Particularly, in the anodizing step, a tantalum substrate is placed in an oxygen-containing electrolyte solution at a temperature of room temperature to 380°C, preferably room temperature to 300°C, and an anode voltage of 3 to 800V is applied, which voltage is maintained for 0.01 to 2 hours, with a boosting current density of 1 to 200 mA/cm², to form a layer of amorphous Ta₂O₅ membrane.

In the anodizing step, if the temperature of the solution is high, the voltage applied should be low; otherwise, it can be high. For example, for a 0.01% H₃PO₄ solution at room temperature, a maximum voltage of 600V can be applied. The voltage applied should be below the flash voltage of the solution regardless of the type of the solution.

In the anodizing step, the oxygen-containing electrolyte solution may be an aqueous solution, a non-aqueous solution, or a mixture of an aqueous electrolyte and an organic compound.

An aqueous oxygen-containing electrolyte may be, for example, an aqueous acid, alkali, salt. The solution temperature is from room temperature (about 25°C) to 95°C, the anode voltage is from 5 to 600V, and the voltage is maintained for 60 to 90 minutes. If the temperature is too high, the moisture will evaporate too fast. When the solution temperature is relatively high, the anode voltage should be relatively low.

A non-aqueous oxygen-containing electrolyte may be an anhydrous concentrated sulfuric acid or a molten salt or a mixture of a molten salt and an alkali, such as potassium nitrate, sodium nitrate, lithium nitrate, or their mixtures with alkali of lithium, sodium, potassium, etc.

Another type of oxygen-containing electrolyte solution may be a mixture of an aqueous electrolyte and an organic compound, such as ethanol, ethylene glycol and n-butanol. The temperature of the aqueous solution should be 95°C or lower, otherwise the water volatilizes rapidly and is difficult to control.

In one embodiment of the present invention, the preparation method of the present invention comprises nitriding or carburizing a tantalum substrate in advance. However, nitriding or carburizing treatment may not be performed either. In order to obtain a relatively high hardness, nitriding or carburizing treatment can be performed.

In a preferred embodiment, an ion nitriding furnace, which is filled with nitrogen and hydrogen in a nitrogen:hydrogen ratio of 2:1 to 1:10, is used with a tantalum substrate as a cathode under the conditions of a furnace temperature of 500 to 1000°C and a furnace pressure of 20 to 2000Pa, for nitriding for 0.5 to 6 hours, so that a nitrided layer is formed on the surface of the tantalum substrate and the hardness of the tantalum substrate after nitriding is controlled between HV180 to 480.

According to one embodiment of the present invention, there is provided a method for preparing a dental implant, comprising:
1) selecting a tantalum rod, or a niobium rod or a tantalum-niobium alloy rod with an appropriate diameter;
2) providing a processed cylindrical or conical implant (including processing external threads and internal pore threads, etc., and providing a connector and an abutment that match the implant);
3) performing an electrochemical coating treatment in lithium-containing salt melt, wherein a white lithium tantalate membrane, a lithium niobate membrane, or a lithium tantalate-lithium niobate mixture membrane may be formed on the tantalum, niobium, or tantalum-niobium alloy implant.

The present invention proposes to replace threaded ribs with suitable pores. The pores can go deep into the inner wall of the implant, i.e., perforations, without affecting the strength of the implant. The advantage of this solution is that it can increase the contact surface between the implant and the human bone and increase the compatibility and firmness of the implant.

Of course, the outer pores may not be perforated and a certain thickness is reserved, so that the inner pores of the implant can be threaded, which facilitates the immobilization of the connector or abutment

According to one embodiment of the present invention, there is provided a method for preparing a dental implant, comprising:
1) providing a dental implant blank and designing punching parameters,
   i.e., determining the size of the cylindrical or conical implant, including the outer diameter of the cylinder and the diameter of the inner pores, expanding the cylinder with pores into a planar view, and designing and determining the size of the pores, the pore spacing or the pore distribution, as well as the punching depth of the pores and the shape of the pores, i.e., a straight pore or a tapered pore;
2) punching pores,
   i.e., punching pores according to design requirements through a method for punching a spinneret plate or a laser;
3) polishing,
   i.e., after punching, the excess material on the surface should be removed by polishing, e.g., upon punching with a laser, there will generally be no excess material around the pores, and polishing is relatively easy;
4) welding the punched and polished tantalum flake with argon arc welding or laser welding into the originally designed cylindrical or conical implant, and performing necessary repairs and processing internal threads, etc.;
5) performing the surface treatment on the porous implant and the abutment by an electrochemical method to form a complex porous membrane corresponding to the implant metal and obtain a coated dental implant with pores.

One may also perform a method for perforating an implant (not claimed), comprising:
1) a perforation inlet, i.e., a pore on the side that is intended to come into contact with a human bone, which has a diameter controlled within the range of 0.08 to 0.30 mm, preferably 0.16 to 0.22 mm;
2) an outlet, i.e., a pore at the end where the pore meets the inner pore of the implant, which has a diameter controlled within the range of 0.28 to 0.06 mm, preferably 0.22 to 0.13 mm, and may be a tapered pore or a straight pore, wherein the tapered pore may be more beneficial to guiding the growth of the human bone into the implant.

According to one embodiment of the present invention, a design of a non-perforated implant is provided to ensure the processing of threads and the like to be processed for the installation of an abutment connector, on the inner wall of the central pores of the dental implant; the pores on the implant should be 0.6 to 0.8 mm, otherwise it will not be perforated so that the inner pores are threaded.

In the context of the present invention, niobium and tantalum-niobium alloy substrates are also applicable to embodiments concerning a tantalum substrate.

Unless otherwise specified, tensile strength and elasticity modulus are tested according to the GB/T22315-2008 standard.

### Examples

The nature and purpose of the present invention will be further understood by examples in conjunction with figures below. It should be understood that these examples are for illustrative purpose only and are not intended to limit the scope of the present invention.

### Example 1

Step 1: The metal tantalum was processed into a dense tantalum flake.

Step 2: An anode voltage of 10V was applied for the tantalum flake in a molten lithium nitrate solution at 570°C, and reaction was carried out at said voltage for 30 minutes, to form a porous lithium tantalate membrane layer on the tantalum flake.

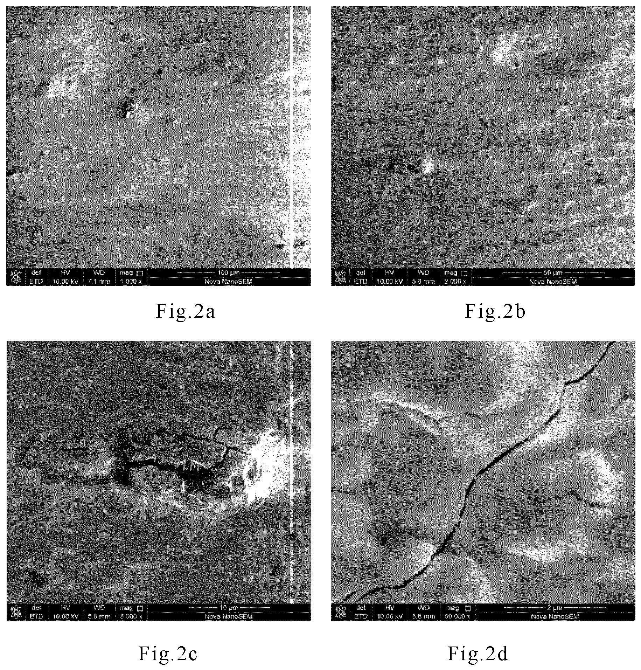

Figures 2a-2d show SEM photographs of the sample of Example 1.

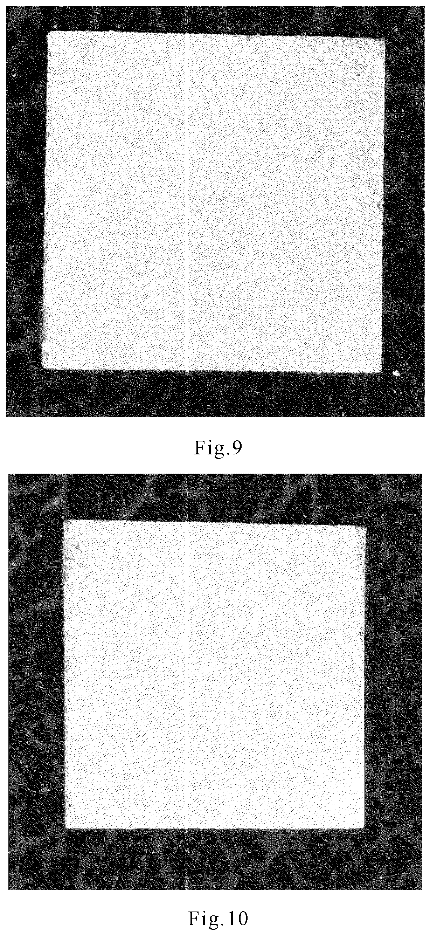

Figure 9 shows an appearance photograph of the sample of Example 1.

### Example 2

Step 1: The metal tantalum was processed into a dense tantalum flake.

Step 2: An anode voltage of 10V was applied for the tantalum flake in a molten lithium nitrate solution at 580°C, and reaction was carried out at said voltage for 25 minutes, to form a porous lithium tantalate membrane layer on the tantalum flake.

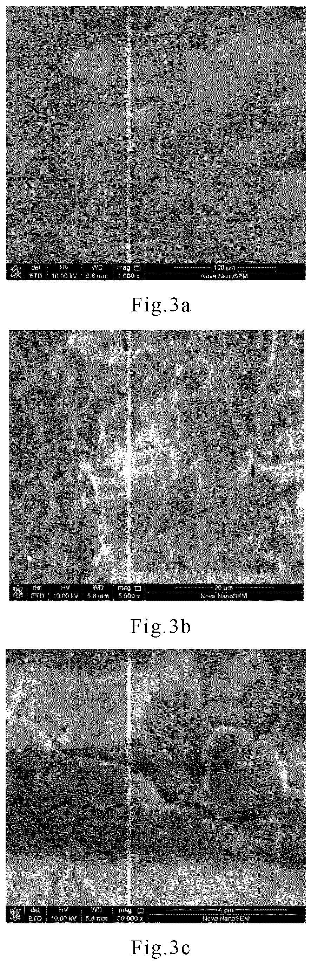

Figures 3a-3c show SEM photographs of the sample of Example 2.

Figure 10 shows an appearance photograph of the sample of Example 2.

### Example 3

Step 1: The metal tantalum was processed into a dense tantalum flake.

Step 2: An anode voltage of 10V was applied for the tantalum flake in a molten lithium nitrate solution at 598°C, and reaction was carried out at said voltage for 10 minutes, to form a porous lithium tantalate membrane layer on the tantalum flake.

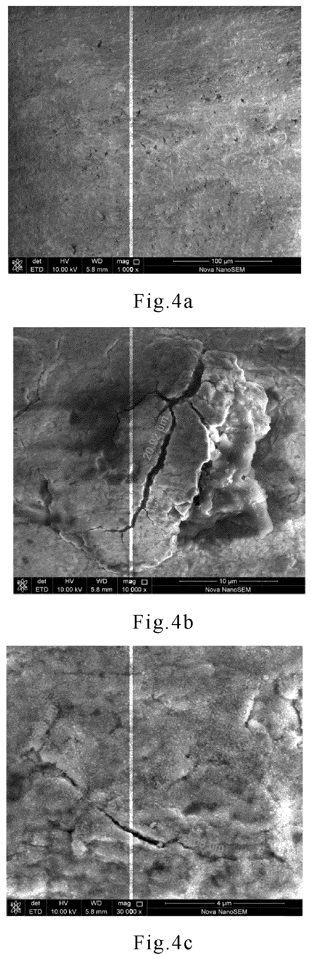

Figures 4a-4c show SEM photographs of the sample of Example 3.

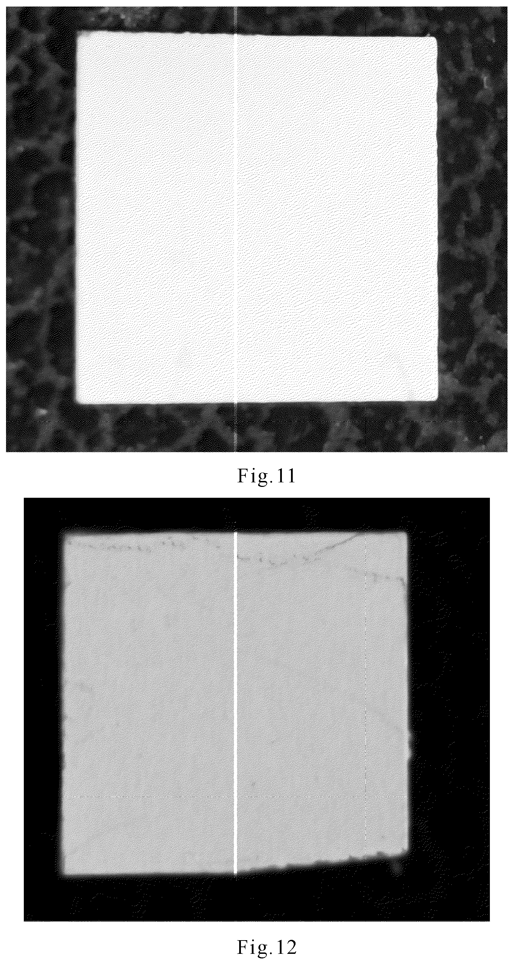

Figure 11 shows an appearance photograph of the sample of Example 3.

### Example 4

Step 1: The metal tantalum was processed into a dense tantalum flake.

Step 2: An anode voltage of 60V was applied for the tantalum flake in a solution having a volume ratio of ethylene glycol:0.01% H₃PO₄ of 2:1 and a temperature of 90°C, and said voltage was held for 3 hours, followed by cleaning.

Step 3: An anode voltage of 10V was applied for the tantalum flake obtained in step (2) in a molten lithium nitrate solution at 598°C, and reaction was carried out at said voltage for 8 minutes, to form a porous lithium tantalate membrane layer on the tantalum flake.

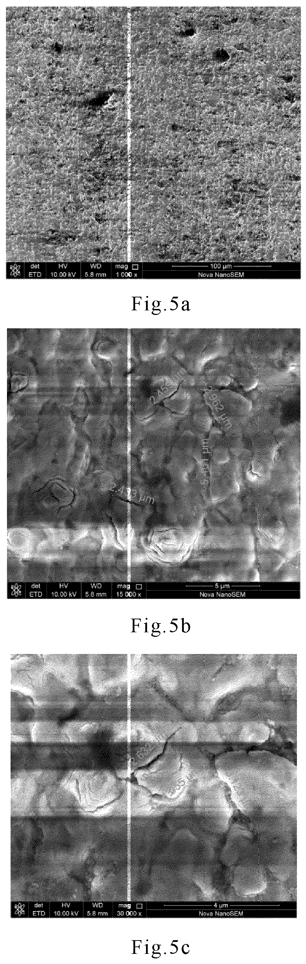

Figures 5a-5c show SEM photographs of the sample of Example 4.

Figure 12 shows an appearance photograph of the sample of Example 4.

### Example 5

Step 1: The metal tantalum was processed into a dense tantalum flake.

Step 2: An anode voltage of 143V was applied for the tantalum flake in a solution having a volume ratio of ethylene glycol:0.01% H₃PO₄ of 2:1 and a temperature of 90°C, and said voltage was held for 3 hours, followed by cleaning.

Step 3: An anode voltage of 28V was applied for the tantalum flake obtained in step (2) in a molten lithium nitrate solution at 482°C, and reaction was carried out at said voltage for 6.5 hours, to form a porous lithium tantalate membrane layer on the tantalum flake.

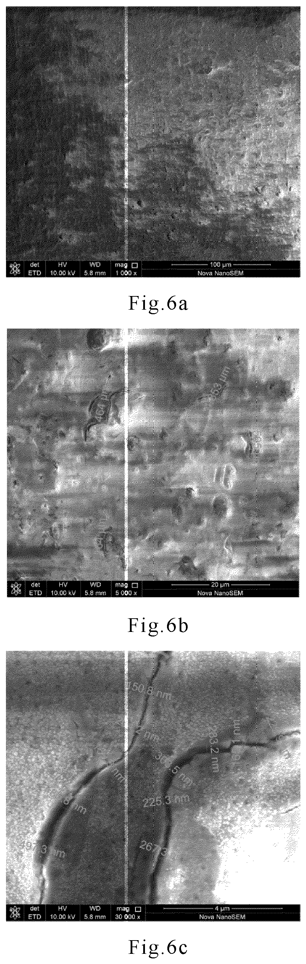

Figures 6a-6c show SEM photographs of the sample of Example 5.

### Example 6

Step 1: The metal tantalum was processed into a dense tantalum flake.

Step 2: An anode voltage of 33V was applied for the tantalum flake in a molten lithium nitrate solution at 440°C, and reaction was carried out at said voltage for 12 hours, to form a porous lithium tantalate membrane layer on the tantalum flake.

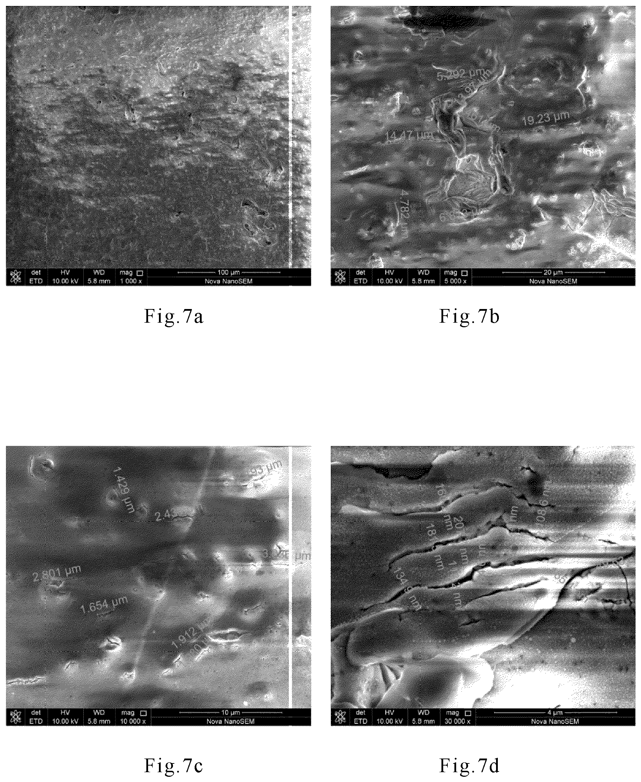

Figures 7a-7d show SEM photographs of the sample of Example 6.

### Example 7

Step 1: The metal tantalum was processed into a dense tantalum flake.

Step 2: An anode voltage of 60V was applied for the tantalum flake in a solution having a volume ratio of ethylene glycol:0.01% H₃PO₄ of 2:1 and a temperature of 90°C, and said voltage was held for 3 hours, followed by cleaning.

Step 3: An anode voltage of 10V was applied for the tantalum flake obtained in step (2) in a molten lithium nitrate solution at 598°C, and reaction was carried out at said voltage for 10 minutes, to form a porous lithium tantalate membrane layer on the tantalum flake.

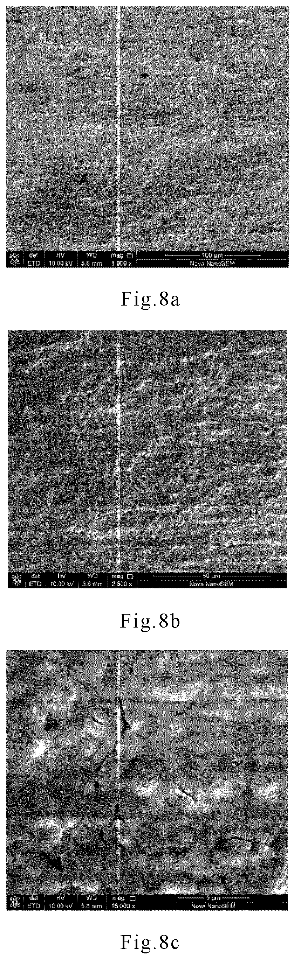

Figures 8a-8c show SEM photographs of the sample of Example 7.

### Example 8

Step 1: The metal tantalum was processed into a dense tantalum flake and a tantalum dental implant, respectively.

Step 2: An anode voltage of 10V was applied for the tantalum flake and the tantalum dental implant (a threaded tantalum rod having a diameter of 6 mm, with pores having a diameter of 2 mm), respectively, in a molten lithium nitrate:potassium nitrate (in a weight ratio of 1:1) solution at 580°C, and reaction was carried out at said voltage for 30 minutes, to form a porous lithium tantalate membrane layer on the tantalum flake and the tantalum dental implant.

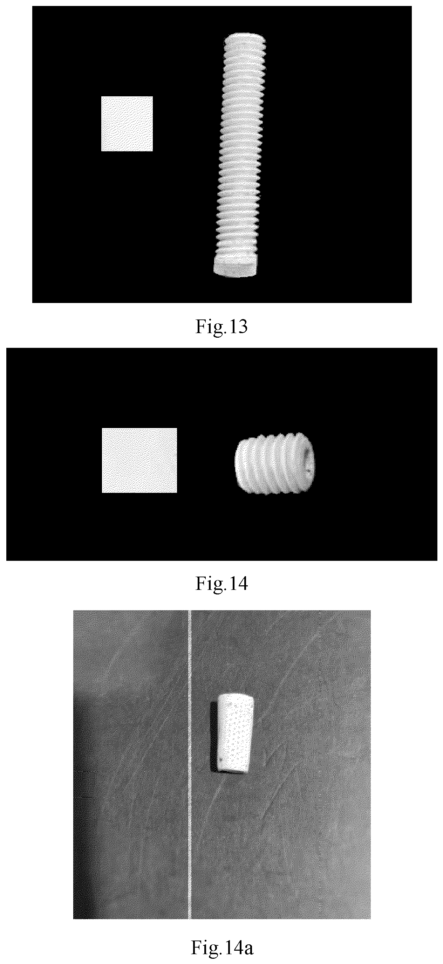

Figure 13 shows an appearance photograph of the sample of Example 8.

### Example 9

Step 1: The metal tantalum was processed into a dense tantalum flake and a tantalum dental implant (a threaded tantalum rod having a diameter of 6 mm, with pores having a diameter of 2 mm), respectively.

Step 2: First of all, an anode voltage of 60V was applied for the tantalum flake and the tantalum dental implant, respectively, in 0.05 wt% of a H₃PO₄ aqueous solution at 90°C, and said voltage was held for 1.5 hours, followed by cleaning.

Step 3: An anode voltage of 10V was applied for the tantalum flake and the tantalum dental implant obtained in step (2) in molten lithium nitrate:potassium nitrate (in a weight ratio of 1:1) at 598°C, and reaction was carried out at said voltage for 8 minutes, to form a porous lithium tantalate membrane layer on the tantalum flake and the tantalum dental implant.

Figure 14 shows an appearance photograph of the sample of Example 9.

### Example 9a

An anode voltage of 10V was applied across a porous tantalum dental implant in molten lithium nitrate: potassium nitrate (in a weight ratio of 1:1) at 580°C, and reaction was carried out at said voltage for 25 minutes.

Figure 14a shows an appearance photograph of the sample of Example 9a.

### Comparative Example 1

The metal tantalum was processed into a dense tantalum flake without the step of forming a porous lithium tantalate membrane layer on the tantalum flake.

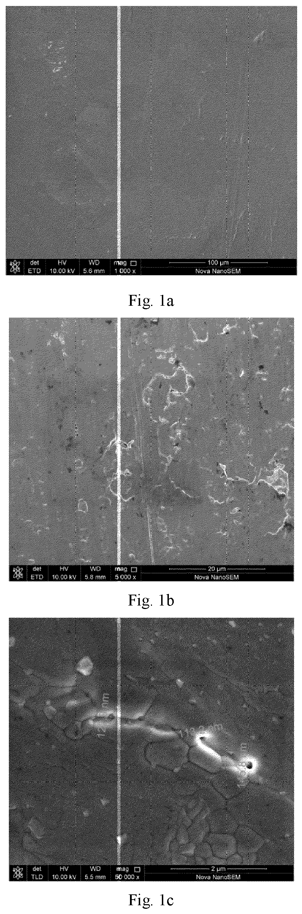

Figures 1a-1c show SEM photographs of the sample of Comparative Example 1.

**Table 1 Tensile Strength of Samples**

| No. | Test Temperature/°C | Size Before Test/mm Thickness *a₀*×Width *b₀* | Original Gauge Length of Sample *L₀*/mm | Maximum Force *Fₘ*/N | Tensile Strength *Rₘ*/MPa |
|---|---|---|---|---|---|
| Example 1 | 25 | 0.322×9.96 | 70 | 1530 | 477 |
| Example 2 | 25 | 0.333×10.21 | 70 | 1478 | 435 |
| Example 3 | 25 | 0.346×10.11 | 70 | 1101 | 315 |
| Example 4 | 25 | 0.341×10.12 | 70 | 1279 | 371 |
| Comparative Example 1 | 25 | 0.332×9.96 | 70 | 822 | 322 |

**Table 2 Elasticity Modulus of Samples**

| No. | Test Temperature/°C | Size Before Test/mm Thickness *a₀*×Width *b₀* | Original Gauge Length of Sample *L₀*/mm | Mass m/g | Dynamic Elasticity Modulus *E_{d}*/GPa |
|---|---|---|---|---|---|
| Example 1 | 25.4 | 5.14×25.19 | 75.34 | 161.288 | 186 |
| Example 2 | 25.4 | 5.17×25.20 | 75.20 | 160.406 | 180 |
| Example 3 | 25.4 | 5.12×25.25 | 75.18 | 159.118 | 181 |
| Example 4 | 25.4 | 5.11×25.19 | 75.25 | 160.062 | 184 |
| Example 5 | 25.4 | 5.17×25.18 | 75.17 | 160.882 | 190 |
| Example 6 | 25.4 | 5.08×25.17 | 75.14 | 160.762 | 189 |
| Comparative Example 1 | 25.4 | 5.10×25.18 | 75.16 | 158.140 | 178 |

| | | | | | |
|---|---|---|---|---|---|
| Note: In Table 2, the coated samples have a membrane thickness of about 10 µm and the transition layers under the membranes are about 30 µm; apparently, the proportion thereof in the thick tantalum test sample is very small; therefore, the measured data are very close to those of pure tantalum. The test data in Table 2 are substantially close to the elasticity modulus of pure tantalum and cannot represent the elasticity modulus of the surface layers applied on the samples in the present invention; however, the values measured by nanoindentation, e.g., with instrument model TI-950, are capable of representing the variations in the elasticity modulus of different coating processes in the present invention. | | | | | |

The data in Table 1 and Table 2 show that as compared to a tantalum flake not coated with a porous lithium tantalate membrane, a tantalum flake coated with a porous lithium tantalate membrane is greatly improved in tensile strength and elasticity modulus, and particularly has an elasticity modulus much greater than the elasticity modulus of 70 GPa required for preparing dental implants, which is very advantageous.

By comparing the SEM photographs of a tantalum flake coated with a porous lithium tantalate membrane and a tantalum flake not coated with a porous lithium tantalate membrane, it can be shown that the tantalum flake coated with a porous lithium tantalate membrane in the present invention has more openings with a pore diameter in the range of about 1-30 µm, the surfaces of which are rough. These pores and uneven rough surfaces are conducive to cell adhesion and tissue mosaicism and can enhance connection strength between bone implants, such as dental implants, and bone tissues, so that the bone implants can make human biological tissues to be bound with tantalum substrates through porous lithium tantalate membranes and the tantalum substrates can be fully and more tightly bound and integrated with human tissues, such as alveolar bones. However, the tantalum flakes that are not coated with porous lithium tantalate membranes have relatively few pores with smooth and flat surfaces, which is not conducive to close integration of tantalum substrates and human tissues, such as alveolar bones.

In addition, from the SEM photographs, it is apparent that the pores of a tantalum flake coated with a porous lithium tantalate membrane have a size in the smallest dimension in the range of about 10 nm to 1 µm, which is much smaller than the size (which is generally greater than 5 µm) of bacteria. Therefore, the bone implant coated with a porous lithium tantalate membrane in the present invention can well inhibit the entry of bacteria, thereby achieving a certain antibacterial effect.

Figures 9-12 show that a tantalum flake coated with a porous lithium tantalate membrane is white, has an aesthetic appearance and is particularly suitable for use in the preparation of dental implants.

### Example 10

A lithium tantalate membrane layer was formed on a tantalum flake in a molten lithium nitrate: potassium nitrate (in a weight ratio of 1:1) solution, and the process conditions for forming the lithium tantalate membrane layer are shown in Table 3.

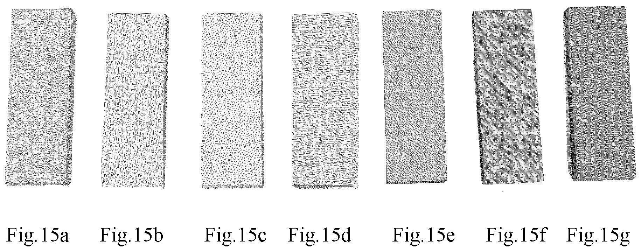

Figure 15a shows an appearance photograph of the sample of Example 10.

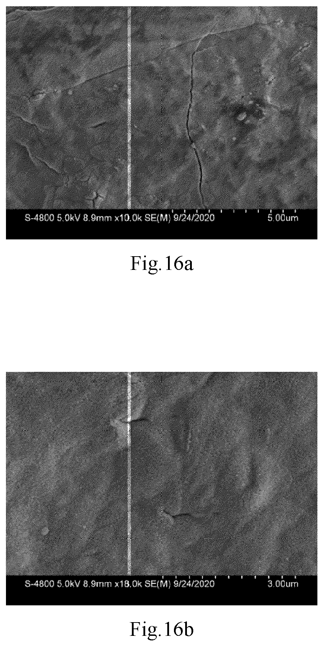

Figures 16a-16b show SEM photographs of the sample of Example 10.

### Example 11

A lithium tantalate membrane layer was formed on a tantalum flake and a tantalum cranium substrate, respectively, in a molten lithium nitrate:potassium nitrate (in a weight ratio of 1:1) solution, and the process conditions for forming the lithium tantalate membrane layer are shown in Table 3.

Figure 15b (a tantalum flake) and Figure 33a (a cranium) show appearance photographs of the sample of Example 11.

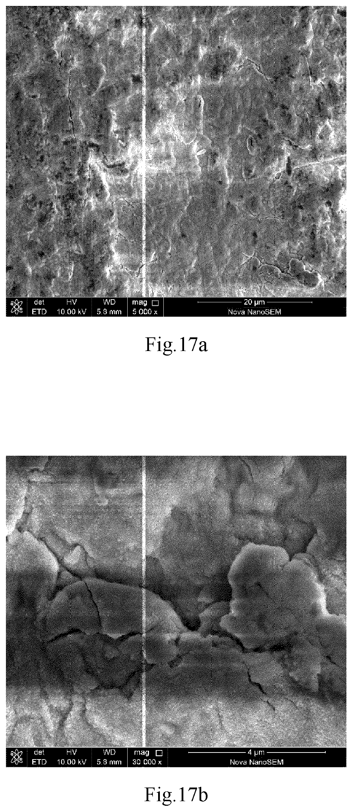

Figures 17a-17b show SEM photographs of the sample of Example 11.

### Example 12

A lithium tantalate membrane layer was formed on a tantalum flake in a molten lithium nitrate: potassium nitrate (in a weight ratio of 1:1) solution, and the process conditions for forming the lithium tantalate membrane layer are shown in Table 3.

Figure 15c shows an appearance photograph of the sample of Example 12.

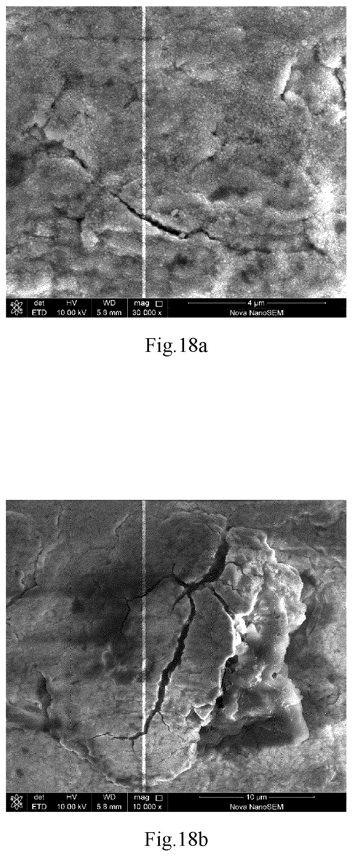

Figures 18a-18b show SEM photographs of the sample of Example 12.

### Example 13

First of all, an anode voltage of 60V was applied on a tantalum flake in a solution having a volume ratio of ethylene glycol:0.01 wt% H₃PO₄ of 2:1 and a temperature of 90°C, and said voltage was held for 1.5 hours, followed by cleaning. After that, a lithium tantalate membrane layer was formed in a molten lithium nitrate:potassium nitrate (in a weight ratio of 1:1) solution, and the process conditions for forming the lithium tantalate membrane layer are shown in Table 3.

Figure 15d shows an appearance photograph of the sample of Example 13.

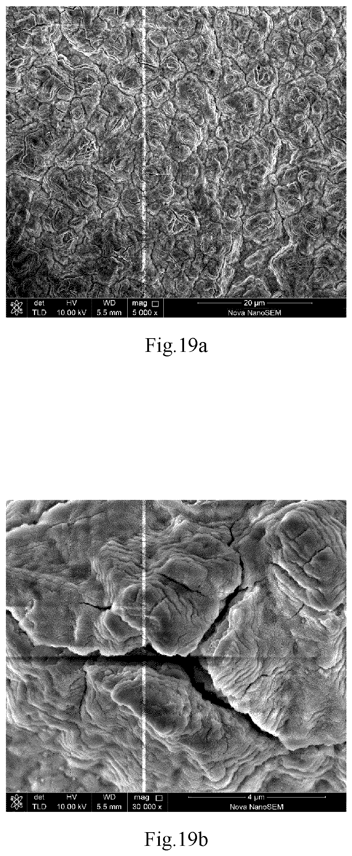

Figures 19a-19b show SEM photographs of the sample of Example 13.

### Example 14

First of all, an anode voltage of 60V was applied on a tantalum flake in a solution having a volume ratio of ethylene glycol:0.01 wt% H₃PO₄ of 2:1 and a temperature of 90°C, and said voltage was held for 1.5 hours, followed by cleaning. After that, a lithium tantalate membrane layer was formed in a molten lithium nitrate:potassium nitrate (in a weight ratio of 1:1) solution, and the process conditions for forming the lithium tantalate membrane layer are shown in Table 3.

Figure 15e shows an appearance photograph of the sample of Example 14.

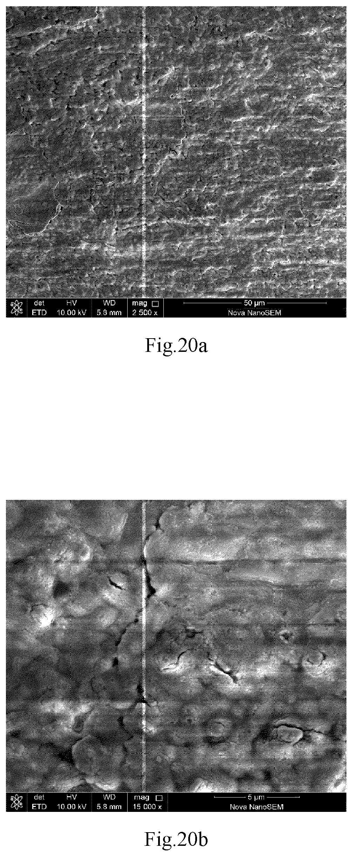

Figures 20a-20b show SEM photographs of the sample of Example 14.

### Example 15

First of all, an anode voltage of 143V was applied on a tantalum flake in a solution having a volume ratio of ethylene glycol:0.01 wt% H₃PO₄ of 2:1 and a temperature of 90°C, and said voltage was held for 1.5 hours, followed by cleaning. After that, a lithium tantalate membrane layer was formed in a molten lithium nitrate:potassium nitrate (in a weight ratio of 1:1) solution, and the process conditions for forming the lithium tantalate membrane layer are shown in Table 3.

Figure 15f shows an appearance photograph of the sample of Example 15.

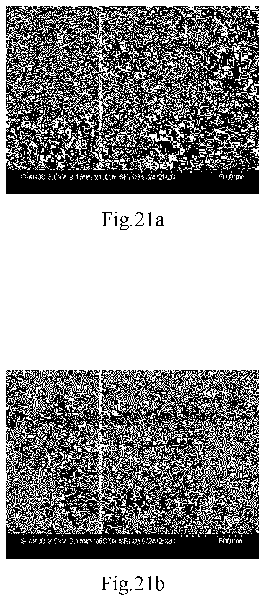

Figures 21a-22b show SEM photographs of the sample of Example 15.

### Example 16

A lithium tantalate membrane layer was formed on a tantalum flake in a molten lithium nitrate: potassium nitrate (in a weight ratio of 1:1) solution, and the process conditions are shown in Table 3.

Figure 15g shows an appearance photograph of the sample of Example 16.

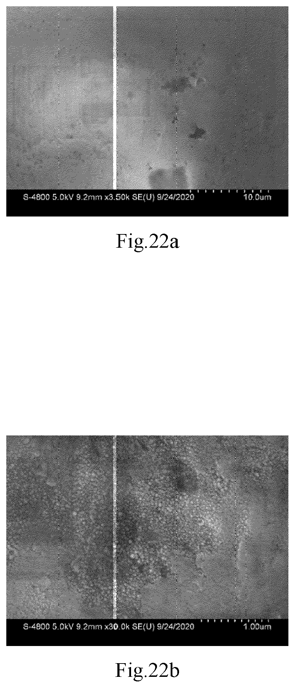

Figures 22a-22b show SEM photographs of the sample of Example 16.

### Example 17

First of all, an anode voltage of 150V was applied on a tantalum flake in a 98% concentrated H₂SO₄ solution at 220°C, and said voltage was held for 30 minutes, followed by cleaning and drying. After that, a lithium tantalate membrane layer was formed in a molten lithium nitrate: potassium nitrate (in a weight ratio of 1:1) solution, and the process conditions for forming the lithium tantalate membrane layer are shown in Table 3.

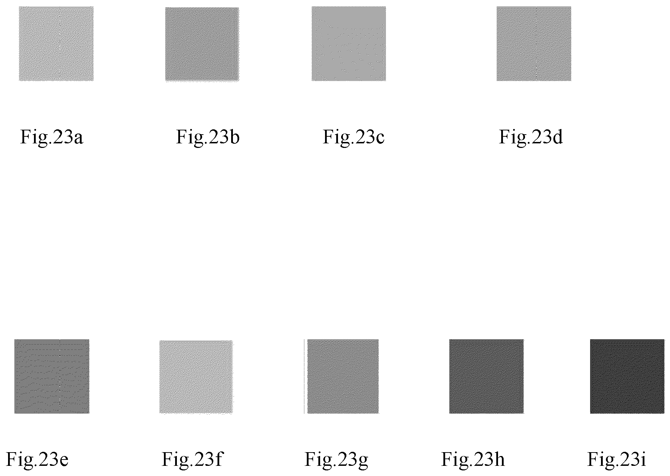

Figure 23a shows an appearance photograph of the sample of Example 17.

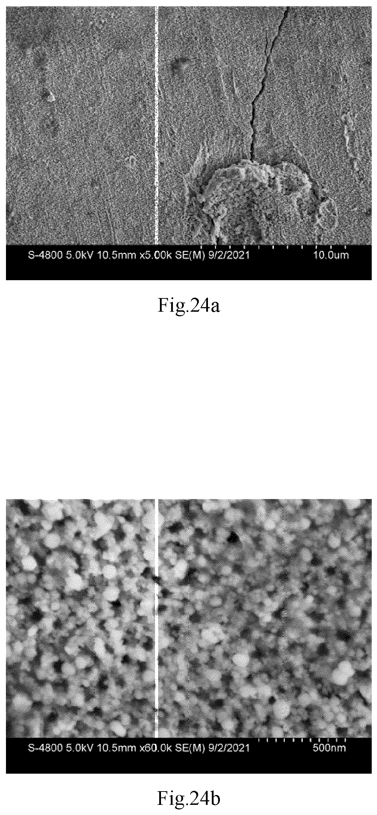

Figures 24a-24b show SEM photographs of the sample of Example 17.

### Example 18

First of all, an anode voltage of 150V was applied on a tantalum flake in a 98% concentrated H₂SO₄ solution at 220°C, and said voltage was held for 30 minutes, followed by cleaning and drying. After that, a lithium tantalate membrane layer was formed in a molten lithium nitrate: potassium nitrate (in a weight ratio of 1:1) solution, and the process conditions for forming the lithium tantalate membrane layer are shown in Table 3.

Figure 23b shows an appearance photograph of the sample of Example 18.

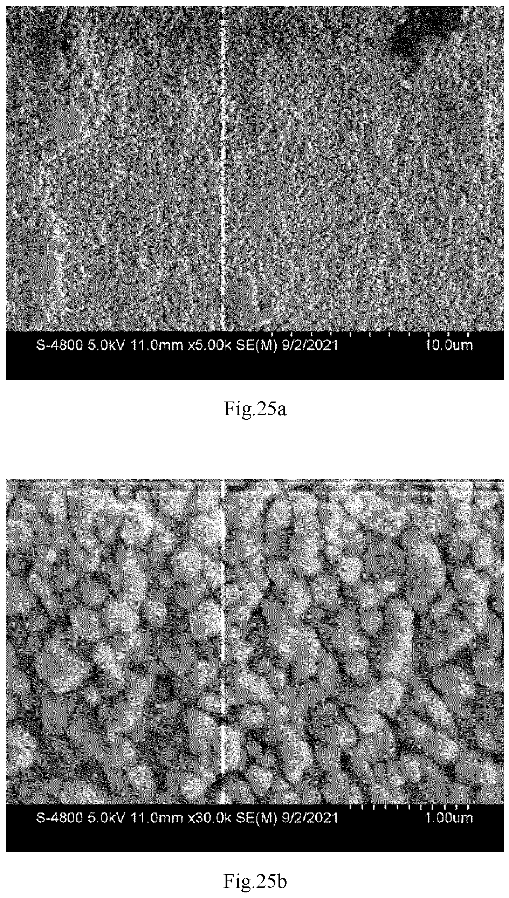

Figures 25a-25b show SEM photographs of the sample of Example 18.

### Example 19

First of all, an anode voltage of 150V was applied on a tantalum flake in a 98% concentrated H₂SO₄ solution at 220°C, and said voltage was held for 30 minutes, followed by cleaning and drying. After that, a lithium tantalate membrane layer was formed in a molten lithium nitrate: potassium nitrate (in a weight ratio of 1:1) solution, and the process conditions for forming the lithium tantalate membrane layer are shown in Table 3.

Figure 23c shows an appearance photograph of the sample of Example 19.

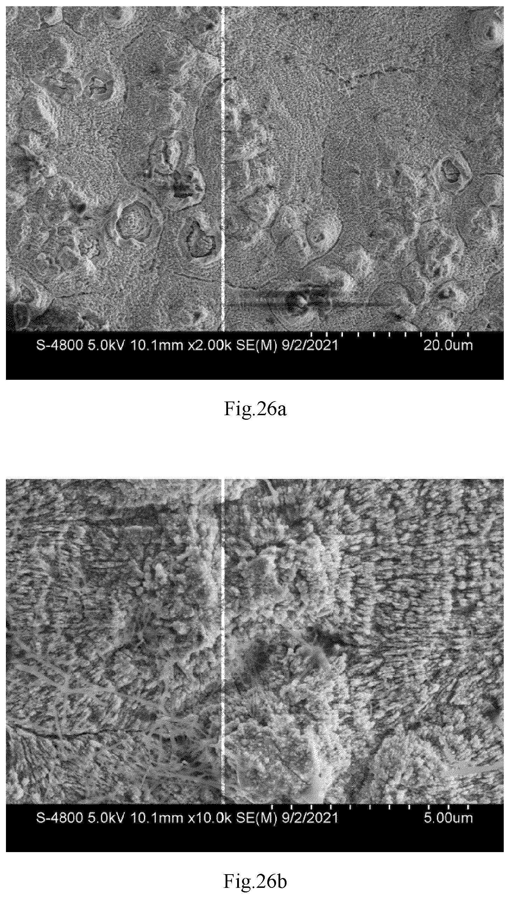

Figures 26a-26b show SEM photographs of the sample of Example 19.

### Example 20

First of all, an anode voltage of 82V was applied on a tantalum flake in a 98% concentrated H₂SO₄ solution at 220°C, and said voltage was held for 30 minutes, followed by cleaning and drying. After that, a lithium tantalate membrane layer was formed in a molten lithium nitrate: potassium nitrate (in a weight ratio of 1:1) solution, and the process conditions for forming the lithium tantalate membrane layer are shown in Table 3.

Figure 23d shows an appearance photograph of the sample of Example 20.

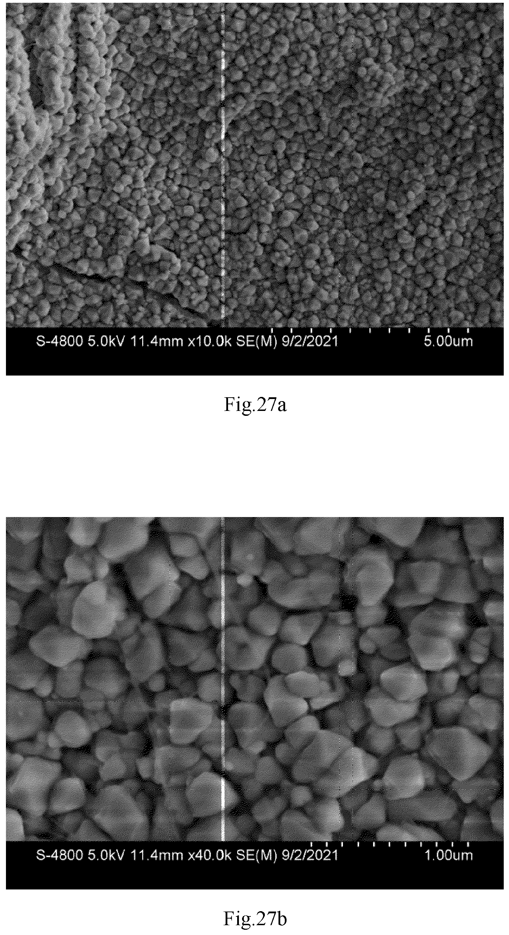

Figures 27a-27b show SEM photographs of the sample of Example 20.

### Example 21

First of all, an anode voltage of 82V was applied on a tantalum flake in a 98% concentrated H₂SO₄ solution at 210°C, and said voltage was held for 30 minutes, followed by cleaning and drying. After that, a lithium tantalate membrane layer was formed in a molten lithium nitrate: potassium nitrate (in a weight ratio of 1:1) solution, and the process conditions for forming the lithium tantalate membrane layer are shown in Table 3.

Figure 23e shows an appearance photograph of the sample of Example 21.

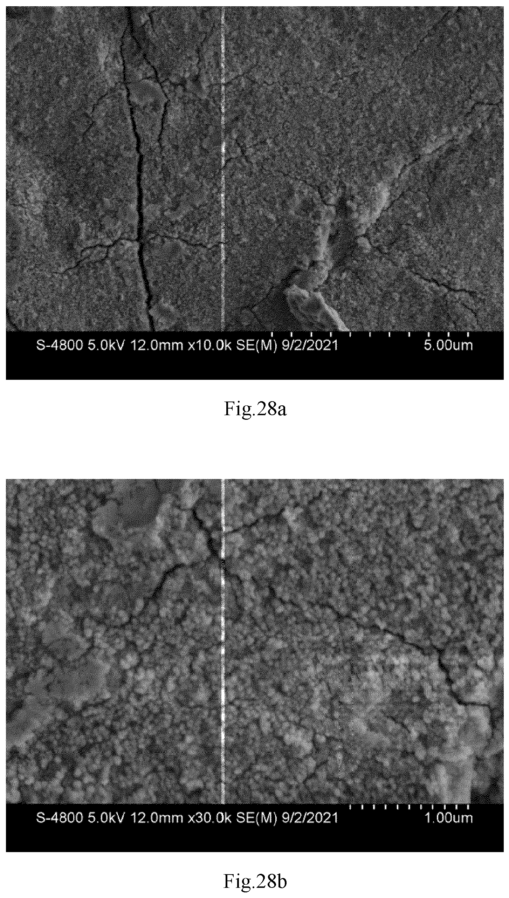

Figures 28a-28b show SEM photographs of the sample of Example 21.

### Example 22

First of all, an anode voltage of 82V was applied on a tantalum flake in a 98% concentrated H₂SO₄ solution at 210°C, and said voltage was held for 30 minutes, followed by cleaning and drying. After that, a lithium tantalate membrane layer was formed in a molten lithium nitrate: potassium nitrate (in a weight ratio of 1:1) solution, and the process conditions for forming the lithium tantalate membrane layer are shown in Table 3.

Figure 23f shows an appearance photograph of the sample of Example 22.

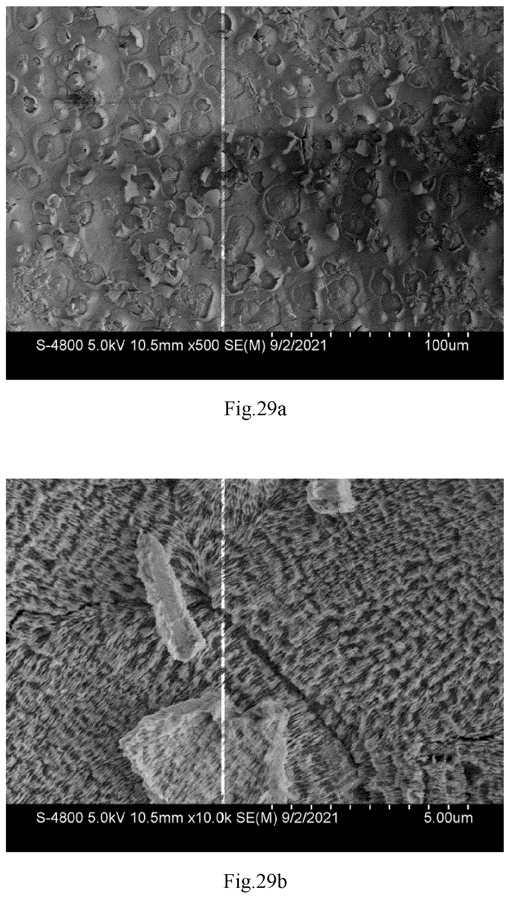

Figures 29a-29b show SEM photographs of the sample of Example 22.

### Example 23

First of all, an anode voltage of 82V was applied on a tantalum flake in a 98% concentrated H₂SO₄ solution at 210°C, and said voltage was held for 30 minutes, followed by cleaning and drying. After that, a lithium tantalate membrane layer was formed in a molten lithium nitrate: potassium nitrate (in a weight ratio of 1:1) solution, and the process conditions for forming the lithium tantalate membrane layer are shown in Table 3.

Figure 23g shows an appearance photograph of the sample of Example 23.

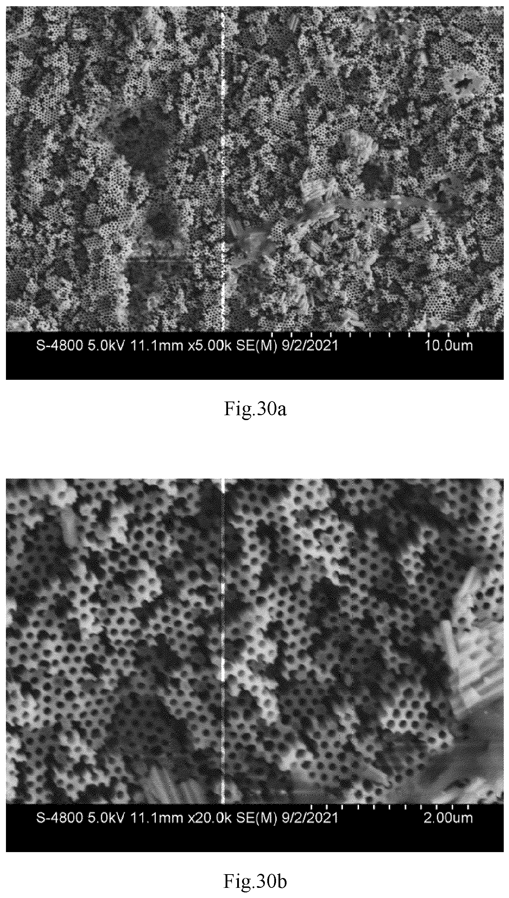

Figures 30a-30b show SEM photographs of the sample of Example 23.

### Example 24

A tantalum flake was anodized in a 98% concentrated H₂SO₄ solution at 220°C, and an anode voltage of 150V was applied, which voltage was maintained for 30 minutes. See Table 3.

Figure 23h shows an appearance photograph of the sample of Example 24.

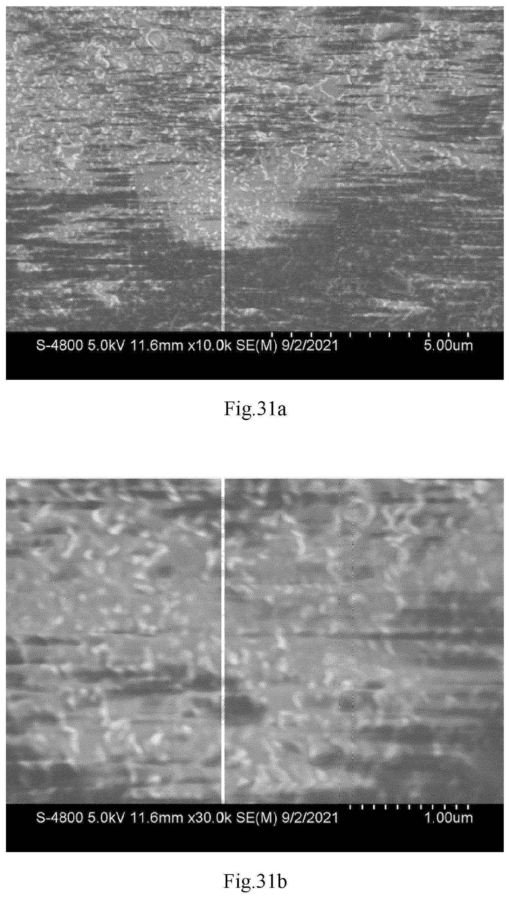

Figures 31a-31b show SEM photographs of the sample of Example 24.

### Example 25 (not according to the invention)

A titanium flake was anodized in a molten lithium nitrate: potassium nitrate (in a weight ratio of 1:1) solution to form a titanium oxide membrane layer, and the process conditions for forming the titanium oxide membrane layer are shown in Table 3.

Figure 23i shows an appearance photograph of the sample of Example 25.

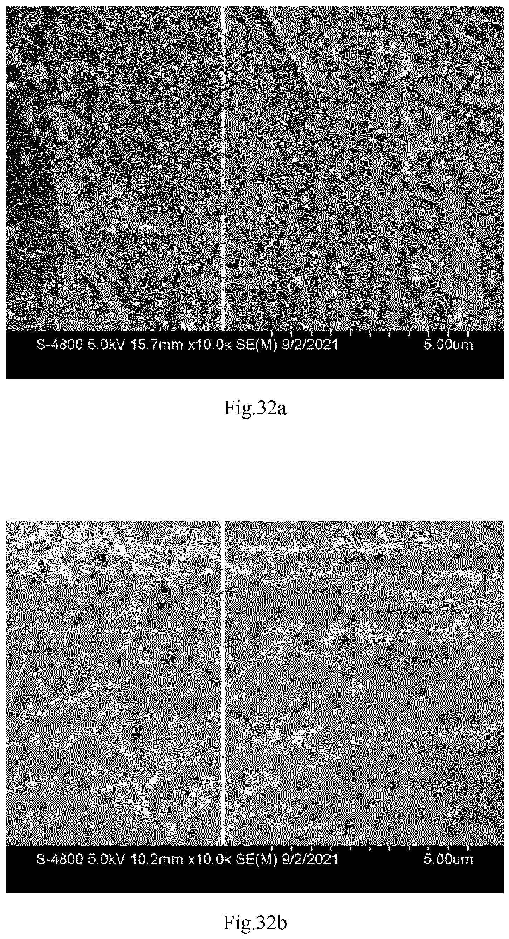

Figures 32a-32b show SEM photographs of the sample of Example 25.

The data in Table 3 show that the tantalum complexes coated with a porous lithium tantalate membrane have a suitable elasticity modulus and are suitable, for example, for the preparation of cranium (skull) implants.

By comparing the SEM photographs of a tantalum flake coated with a porous lithium tantalate membrane and a tantalum flake not coated with a porous lithium tantalate membrane, it can be shown that the tantalum flake coated with a porous lithium tantalate membrane in the present invention has more openings with a pore diameter in the range of about 0.1-30 µm and even honeycomb openings, the surfaces of which are rough. These pores and uneven rough surfaces are conducive to cell adhesion and tissue mosaicism and can enhance connection strength between bone implants, such as dental implants, and bone tissues, such as craniums (skulls), so that the bone implants can make human biological tissues to be bound with tantalum substrates through porous lithium tantalate membranes and the tantalum substrates can be fully and more tightly bound and integrated with human tissues. However, the tantalum flakes that are not coated with porous lithium tantalate membranes have relatively few pores with smooth and flat surfaces, which is not conducive to close integration of tantalum substrates and human tissues, such as alveolar bones and craniums (skulls).

The titanium flake with a titanium oxide membrane layer prepared in Example 25 exhibits a suitable elasticity modulus and its SEM photographs show that the membrane layer has a fibrous porous interactive network (see Figure 32b), which is conducive to cell adhesion and tissue mosaicism and can enhance connection strength between bone implants and bone tissues (such as craniums (skulls)), so that the bone implants can make human biological tissues to be bound with titanium substrates through porous titanium oxide membrane layers and the titanium substrates can be fully and more tightly bound and integrated with human tissues.

In addition, from the SEM photographs, it is apparent that the pores of a tantalum flake coated with a porous lithium tantalate membrane have a size in the smallest dimension in the range of about 10 nm to 1 µm, which is much smaller than the size (which is generally greater than 5 µm) of bacteria. Therefore, the bone implant coated with a porous lithium tantalate membrane in the present invention can well inhibit the entry of bacteria, thereby achieving a certain antibacterial effect.

### Example 26

A lithium tantalate-lithium niobate mixture membrane layer was formed on a tantalum-niobium alloy flake in a molten lithium nitrate: potassium nitrate (in a weight ratio of 1:1) solution, and the process conditions for forming the lithium tantalate-lithium niobate mixture membrane layer are shown in Table 3.

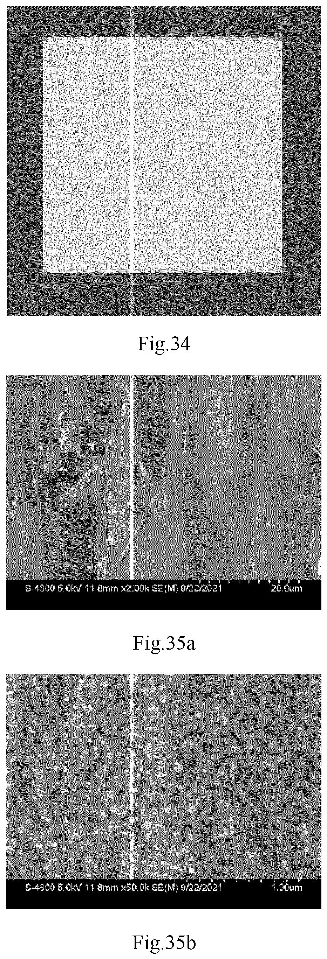

Figure 34 shows an appearance photograph of the sample of Example 26.

Figures 35a-35b show SEM photographs of the sample of Example 26.

### Example 27

First of all, an anode voltage of 143V was applied on a tantalum-niobium alloy flake in a solution having a volume ratio of ethylene glycol:0.01 wt% H₃PO₄ of 2:1 and a temperature of 90°C, and said voltage was held for 1.5 hours, followed by cleaning. After that, a lithium tantalate-lithium niobate mixture membrane layer was formed in a molten lithium nitrate:potassium nitrate (in a weight ratio of 1:1) solution, and the process conditions for forming the lithium tantalate-lithium niobate mixture membrane layer are shown in Table 3.

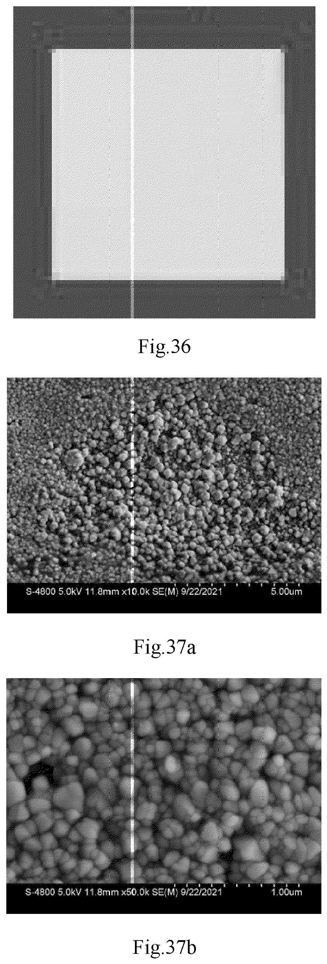

Figure 36 shows an appearance photograph of the sample of Example 27.

Figures 37a-37b show SEM photographs of the sample of Example 27.

### Example 28

A lithium niobate membrane layer was formed on a niobium flake in a molten lithium nitrate: potassium nitrate (in a weight ratio of 1:1) solution, and the process conditions for forming the lithium niobate membrane layer are shown in Table 3.

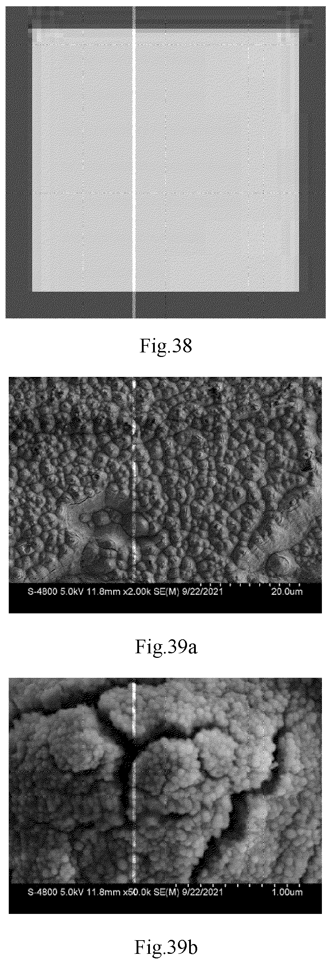

Figure 38 shows an appearance photograph of the sample of Example 28.

Figures 39a-39b show SEM photographs of the sample of Example 28.

### Example 29

A lithium niobate membrane layer was formed on a niobium flake and a niobium cranium substrate in a molten lithium nitrate:potassium nitrate (in a weight ratio of 1:1) solution, and the process conditions for forming the lithium niobate membrane layer are shown in Table 3.

Figure 40 (a niobium flake) and Figure 41 (a niobium cranium) show appearance photographs of the sample of Example 29.

Figures 42a-42b show SEM photographs of the sample of Example 29.

### Example 30

A lithium niobate membrane layer was formed on a niobium flake in a molten lithium nitrate: potassium nitrate (in a weight ratio of 1:1) solution, and the process conditions for forming the lithium niobate membrane layer are shown in Table 3.

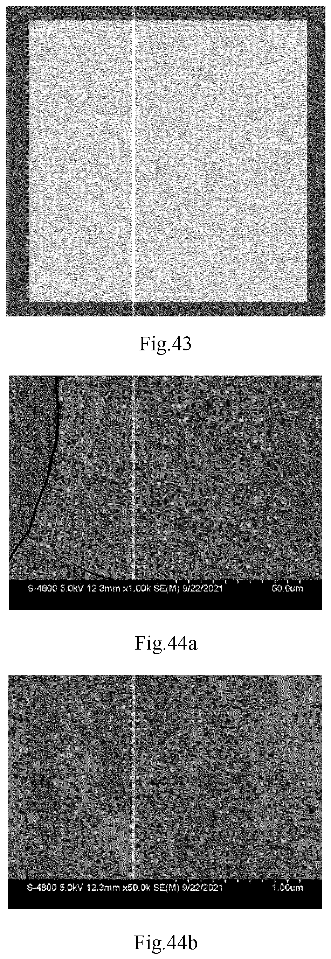

Figure 43 shows an appearance photograph of the sample of Example 30.

Figures 44a-44b show SEM photographs of the sample of Example 30.

**Table 3 Preparation and Elasticity Modulus of Samples (measured by nanoindenter TI-950, NHT, from Hysitron, US)**

| No. | Test Temperature (°C) | Test Voltage (V) | Test Time (min) | Elasticity Modulus (Ed/GPa) | Specification (mm) |
|---|---|---|---|---|---|
| Example 10 | 570 | 10 | 30 | 134.164 | 80*25*5 |
| Example 11 | 580 | 10 | 25 | 43.266 | 80*25*5 |
| Example 12 | 598 | 10 | 10 | 70.108 | 80*25*5 |
| Example 13 | 598 | 10 | 8 | 105.314 | 80*25*5 |
| Example 14 | 598 | 10 | 10 | 87.743 | 80*25*5 |
| Example 15 | 482 | 28 | 390 | 74.678 | 80*25*5 |
| Example 16 | 440 | 33 | 720 | 119.325 | 80*25*5 |
| Example 17 | 482 | 28 | 390 | 93.23964 | 10*10*0.28 |
| Example 18 | 580 | 10 | 25 | 112.601 | 10*10*0.28 |
| Example 19 | 598 | 10 | 10 | 134.1445 | 10*10*0.28 |
| Example 20 | 580 | 10 | 25 | 17.30241 | 10*10*0.28 |
| Example 21 | 482 | 20 | 390 | 115.0177 | 10*10*0.28 |
| Example 22 | 482 | 8 | 390 | 83.64055 | 10*10*0.28 |
| Example 23 | 210 | 82 | 30 | 13.34679 | 10*10*0.28 |
| Example 24 | 220 | 150 | 30 | 24.87508 | 10*10*0.28 |
| Example 25 | 580 | 10 | 110 | 65.62898 | 10*10*0.28 |
| Example 26 | 482 | 28 | 300 | 64.466 | 10*10*0.28 |
| Example 27 | 482 | 28 | 180 | 50.504 | 10*10*0.28 |
| Example 28 | 460 | 28 | 0.083 | 70.108 | 10*10*0.28 |
| Example 29 | 460 | 10 | 20 | 48.356 | 10*10*0.28 |
| Example 30 | 460 | 20 | 5 | 54.295 | 10*10*0.05 |

The elasticity moduli in Table 3 are measured by nanoindenter TI-950, NHT, from Hysitron, US.

The data in Table 3 show that the complexes coated with a porous lithium tantalate-lithium niobate mixture membrane and the complexes with a porous lithium niobate membrane have a suitable elasticity modulus and are suitable, for example, for the preparation of cranium (skull) implants.

Although the specific embodiments of the present invention have been shown and described, it should be understood that other modifications, substitutions and alternatives are known to those skilled in the art. Such modifications, substitutions and alternatives can be made without departing from the scope of the present invention, which should be determined by the attached claims. The various features of the present invention are described in the attached claims.

## Claims

1. A bone implant comprising:
(1) a substrate; and
(2) a porous membrane on the substrate,
wherein one of these features applies:
i) the substrate is a tantalum substrate and the porous membrane is a porous lithium tantalate membrane,
ii) the substrate is a niobium substrate and the porous membrane is a porous lithium niobate membrane, or
iii) the substrate is a tantalum-niobium alloy substrate and the porous membrane is a porous lithium tantalate-lithium niobate mixture membrane.

2. The bone implant according to claim 1,
wherein the porous lithium tantalate membrane contains a tantalum oxide;
wherein the porous lithium niobate membrane contains a niobium oxide; and
wherein the porous lithium tantalate-lithium niobate mixture membrane contains a tantalum oxide and a niobium oxide.

3. The bone implant according to claim 1, wherein the porous membrane has a pore diameter of 0.1-1 µm.

4. The bone implant according to claim 1, wherein the porous membrane has a thickness of 1-20 µm.

5. The bone implant according to claim 1, wherein the bone implant has an elasticity modulus of at least 150 GPa or an elasticity modulus of 30-140 GPa.

6. The bone implant according to claim 1, wherein the bone implant comprises a bone prosthesis, a dental implant and a cranium.

7. The bone implant according to claim 1, wherein the tantalum substrate, the niobium substrate or the tantalum-niobium alloy substrate is porous or dense.

8. A method for preparing a bone implant according to claim 1, comprising:
(1) providing a substrate; and
(2) forming a porous membrane on the substrate,
wherein one of these features applies:
(i) the substrate is a tantalum substrate and the porous membrane is a porous lithium tantalate membrane, which contains a tantalum oxide,
(ii) the substrate is a niobium substrate and the porous membrane is a porous lithium niobate membrane, which contains a niobium oxide or
(iii) the substrate is a tantalum-niobium alloy substrate and the porous membrane is a porous lithium tantalate-lithium niobate mixture membrane, which contains a tantalum oxide and a niobium oxide.

9. The method according to claim 8, wherein in step (2), the porous membrane is formed by a molten lithium salt electrochemical method.

10. The method according to claim 8,
wherein in feature (i), an amorphous Ta₂O₅ membrane is formed on a tantalum substrate by anodic oxidation prior to step (2),
wherein in feature (ii), an amorphous Nb₂O₅ membrane is formed on a niobium substrate by anodic oxidation prior to step (2), and
wherein in feature (iii), an amorphous Ta₂O₅-Nb₂O₅ mixture membrane is formed on a tantalum-niobium alloy substrate by anodic oxidation prior to step (2).

11. A use of a bone implant in a medical material, wherein the bone implant is as defined in claim 1 to 7, or obtainable by a method according to claim 8 - 10.

12. The use according to claim 11, wherein the medical material is a dental implant or a cranium.

13. A method for preparing a dental implant having pores, comprising:
1) providing a dental implant tantalum blank and designing punching parameters,
i.e., determining the size of the cylindrical or conical implant, including the outer diameter of the cylinder and the diameter of the inner pores, expanding the cylinder with pores into a planar view, and designing and determining the size of the pores, the pore spacing or the pore distribution, as well as the punching depth of the pores and the shape of the pores, i.e., a straight pore or a tapered pore,
2) forming a punched tantalum flake by punching pores into the tantalum blank,
3) polishing the punched tantalum flake,
4) welding the punched and polished tantalum flake with argon arc welding or laser welding into the originally designed cylindrical or conical implant, and performing necessary repairs and processing internal threads;
5) performing the surface treatment on the porous implant and an abutment by an electrochemical method to form a complex porous membrane corresponding to the implant metal and obtain a coated dental implant with pores, wherein the porous membrane is a porous lithium tantalate.

14. A non-perforated implant, wherein the inner pores on the implant are 0.6 to 0.8 mm, wherein the implant is a bone implant according to claim 1.

## Patentansprüche

1. Ein Knochenimplantat, umfassend:
(1) ein Substrat; und
(2) eine poröse Membran auf dem Substrat,
wobei eines der folgenden Merkmale zutrifft:
i) das Substrat ein Tantal-Substrat ist und die poröse Membran eine poröse Lithiumtantalat-Membran ist,
ii) das Substrat ein Niob-Substrat ist und die poröse Membran eine poröse Lithiumniobat-Membran ist, oder
iii) das Substrat ein Substrat aus einer Tantal-Niob-Legierung ist und die poröse Membran eine poröse Membran aus einer Lithiumtantalat-Lithiumniobat-Mischung ist.

2. Das Knochenimplantat nach Anspruch 1,
wobei die poröse Lithiumtantalat-Membran ein Tantaloxid enthält;
wobei die poröse Lithiumniobat-Membran ein Nioboxid enthält; und
wobei die poröse Lithiumtantalat-Lithiumniobat-Mischmembran ein Tantaloxid und ein Nioboxid enthält.

3. Das Knochenimplantat nach Anspruch 1, wobei die poröse Membran einen Porendurchmesser von 0,1-1 µm aufweist.

4. Das Knochenimplantat nach Anspruch 1, wobei die poröse Membran eine Dicke von 1 bis 20 µm aufweist.

5. Das Knochenimplantat nach Anspruch 1, wobei das Knochenimplantat einen Elastizitätsmodul von mindestens 150 GPa oder einen Elastizitätsmodul von 30 bis 140 GPa aufweist.

6. Das Knochenimplantat nach Anspruch 1, wobei das Knochenimplantat eine Knochenprothese, ein Zahnimplantat und einen Schädel umfasst.

7. Das Knochenimplantat nach Anspruch 1, wobei das Tantal-Substrat, das Niob-Substrat oder das Tantal-Niob-Legierungs-Substrat porös oder dicht ist.

8. Ein Verfahren zur Herstellung eines Knochenimplantats nach Anspruch 1, umfassend:
(1) Bereitstellen eines Substrats; und
(2) Bilden einer porösen Membran auf dem Substrat,
wobei eines der folgenden Merkmale zutrifft:
(i) das Substrat ein Tantal-Substrat ist und die poröse Membran eine poröse Lithiumtantalat-Membran ist, die Tantaloxid enthält,
(ii) das Substrat ein Niob-Substrat ist und die poröse Membran eine poröse Lithiumniobat-Membran ist, die ein Nioboxid enthält, oder
(iii) das Substrat ein Tantal-Niob-Legierungssubstrat ist und die poröse Membran eine poröse Lithiumtantalat-Lithiumniobat-Mischmembran ist, die ein Tantaloxid und ein Nioboxid enthält.

9. Das Verfahren nach Anspruch 8, wobei in Schritt (2) die poröse Membran durch ein elektrochemisches Verfahren mit geschmolzenem Lithiumsalz gebildet wird.

10. Das Verfahren nach Anspruch 8,
wobei in Merkmal (i) vor Schritt (2) durch anodische Oxidation eine amorphe Ta₂O₅-Membran auf einem Tantal-Substrat gebildet wird,
wobei in Merkmal (ii) vor Schritt (2) durch anodische Oxidation eine amorphe Nb₂O₅-Membran auf einem Niobsubstrat gebildet wird, und
wobei in Merkmal (iii) vor Schritt (2) durch anodische Oxidation eine amorphe Ta₂O₅-Nb₂O₅-Mischmembran auf einem Tantal-Niob-Legierungssubstrat gebildet wird.

11. Eine Verwendung eines Knochenimplantats in einem medizinischen Material, wobei das Knochenimplantat gemäß den Ansprüchen 1 bis 7 definiert ist oder durch ein Verfahren gemäß den Ansprüchen 8 bis 10 erhältlich ist.

12. Die Verwendung gemäß Anspruch 11, wobei das medizinische Material ein Zahnimplantat oder ein Schädel ist.

13. Ein Verfahren zur Herstellung eines Zahnimplantats mit Poren, umfassend:
1) Bereitstellung eines Tantal-Rohlings für ein Zahnimplantat und Festlegung der Stanzparameter,
d. h. Bestimmung der Größe des zylindrischen oder konischen Implantats, einschließlich des Außendurchmessers des Zylinders und des Durchmessers der inneren Poren, Abbildung des Zylinders mit Poren in einer planaren Ansicht sowie Auslegung und Bestimmung der Größe der Poren, des Porenabstands oder der Porenverteilung sowie der Stanztiefe der Poren und der Form der Poren, d. h. einer geraden Pore oder einer konischen Pore,
2) Herstellen eines gestanzten Tantalflakes durch Stanzen von Poren in den Tantalrohling,
3) Polieren des gestanzten Tantalflakes,
4) das Stanzen und Polieren des Tantalflakes mittels Argon-Lichtbogenschweißen oder Laserschweißen zu dem ursprünglich entworfenen zylindrischen oder konischen Implantat und das Durchführen notwendiger Reparaturen sowie das Bearbeiten von Innengewinden;
5) Durchführung der Oberflächenbehandlung des porösen Implantats und eines Abutments mittels eines elektrochemischen Verfahrens, um eine dem Implantatmetall entsprechende komplexe poröse Membran zu bilden und ein beschichtetes Zahnimplantat mit Poren zu erhalten, wobei die poröse Membran ein poröses Lithiumtantalat ist.

14. Ein nicht perforiertes Implantat, wobei die inneren Poren des Implantats 0,6 bis 0,8 mm betragen, wobei das Implantat ein Knochenimplantat gemäß Anspruch 1 ist.

## Revendications

1. Un implant osseux comprenant :
(1) un substrat ; et
(2) une membrane poreuse sur le substrat,
dans lequel l'une des caractéristiques suivantes s'applique :
i) le substrat est un substrat en tantale et la membrane poreuse est une membrane poreuse en tantalate de lithium,
ii) le substrat est un substrat en niobium et la membrane poreuse est une membrane poreuse en niobate de lithium, ou
iii) le substrat est un substrat en alliage de tantale et de niobium et la membrane poreuse est une membrane poreuse constituée d'un mélange de tantalate de lithium et de niobate de lithium.

2. L'implant osseux selon la revendication 1,
dans lequel la membrane poreuse en tantalate de lithium contient de l'oxyde de tantale ;
dans lequel la membrane poreuse en niobate de lithium contient un oxyde de niobium ; et
dans lequel la membrane poreuse en mélange de tantalate de lithium et de niobate de lithium contient un oxyde de tantale et un oxyde de niobium.

3. L'implant osseux selon la revendication 1, dans lequel la membrane poreuse présente un diamètre de pores de 0,1 à 1 µm.

4. L'implant osseux selon la revendication 1, dans lequel la membrane poreuse a une épaisseur de 1 à 20 µm.

5. L'implant osseux selon la revendication 1, dans lequel l'implant osseux présente un module d'élasticité d'au moins 150 GPa ou un module d'élasticité compris entre 30 et 140 GPa.

6. L'implant osseux selon la revendication 1, dans lequel l'implant osseux comprend une prothèse osseuse, un implant dentaire et un crâne.

7. L'implant osseux selon la revendication 1, dans lequel le substrat en tantale, le substrat en niobium ou le substrat en alliage de tantale-niobium est poreux ou dense.

8. Un procédé de préparation d'un implant osseux selon la revendication 1, comprenant :
(1) la fourniture d'un substrat ; et
(2) la formation d'une membrane poreuse sur le substrat,
dans lequel l'une des caractéristiques suivantes s'applique :
(i) le substrat est un substrat en tantale et la membrane poreuse est une membrane poreuse en tantalate de lithium, qui contient de l'oxyde de tantale,
(ii) le substrat est un substrat en niobium et la membrane poreuse est une membrane poreuse en niobate de lithium, qui contient de l'oxyde de niobium, ou
(iii) le substrat est un substrat en alliage de tantale et de niobium et la membrane poreuse est une membrane poreuse constituée d'un mélange de tantalate de lithium et de niobate de lithium, qui contient un oxyde de tantale et un oxyde de niobium.

9. Le procédé selon la revendication 8, dans lequel, à l'étape (2), la membrane poreuse est formée par un procédé électrochimique à base de sel de lithium fondu.

10. Le procédé selon la revendication 8,
dans lequel, dans la caractéristique (i), une membrane amorphe de Ta₂O₅ est formée sur un substrat de tantale par oxydation anodique avant l'étape (2),
dans lequel, dans la caractéristique (ii), une membrane amorphe de Nb₂O₅ est formée sur un substrat de niobium par oxydation anodique avant l'étape (2), et
dans laquelle, dans la caractéristique (iii), une membrane amorphe constituée d'un mélange de Ta₂O₅ et de Nb₂O₅ est formée sur un substrat en alliage de tantale-niobium par oxydation anodique avant l'étape (2).

11. Une utilisation d'un implant osseux dans un matériau médical, dans laquelle l'implant osseux est tel que défini dans les revendications 1 à 7, ou peut être obtenu par un procédé selon les revendications 8 à 10.

12. L'utilisation selon la revendication 11, dans laquelle le matériau médical est un implant dentaire ou un crâne.

13. Un procédé de préparation d'un implant dentaire comportant des pores, comprenant :
1) fournir une ébauche d'implant dentaire en tantale et définir les paramètres de poinçonnage, c'est-à-dire déterminer la taille de l'implant cylindrique ou conique, y compris le diamètre extérieur du cylindre et le diamètre des pores intérieurs, développer le cylindre à pores en une vue en plan, et concevoir et déterminer la taille des pores, l'espacement ou la répartition des pores, ainsi que la profondeur de poinçonnage des pores et la forme des pores, c'est-à-dire un pore droit ou un pore conique,
2) former une lamelle de tantale perforée en perforant des pores dans l'ébauche de tantale,
3) polir la lamelle de tantale perforée,
4) souder la lamelle de tantale perforée et polie par soudage à l'arc sous argon ou par soudage au laser pour former l'implant cylindrique ou conique initialement conçu, et effectuer les réparations nécessaires ainsi que l'usinage des filetages internes ;
5) réaliser le traitement de surface de l'implant poreux et d'un pilier par une méthode électrochimique afin de former une membrane poreuse complexe correspondant au métal de l'implant et d'obtenir un implant dentaire revêtu comportant des pores, dans lequel la membrane poreuse est un tantalate de lithium poreux.

14. Un implant non perforé, dans lequel les pores internes de l'implant mesurent de 0,6 à 0,8 mm, l'implant étant un implant osseux selon la revendication 1.
